# EUROPEAN PATENT APPLICATION

(11) **EP 1 990 639 A1**
(43) Date of publication of application: **12.11.2008**
(21) Application number: 08161973.6
(22) Date of filing: 28.02.2005
(51) Int. Cl.: G01N 33/543, G01N 33/53, A61K 9/51, G01N 33/50, G01N 33/497, A61B 5/11

(54) **System and method for real-time diagnosis, treatment, and therapeutic drug monitoring**

(30) Priority: 26.02.2004 US 788501
(62) Divisional of application: 05756623.4
(71) Applicant: University of Florida Research Foundation, Inc., Gainesville, FL 32611 (US)
(72) Inventor: Melker, Richard J., Gainesville, FL 32605 (US); Sackellares, James Chris, Gainesville, FL 32608 (US); Gold, Mark S., Gainesville, FL 32608 (US); Dennis, Donn Michael, Gainesville, FL 32608-4189 (US); Martin, Charles R., Gainesville, FL 32605 (US); Stewart, Jon D., Gainesville, FL 32606 (US)
(74) Representative: Perry, Robert Edward

(57) **Abstract**

Systems and methods for diagnosing and/or treating diseases as well as monitoring disease treatment. For diagnosis, the present invention uses nanoparticle-based assemblies, which comprise a nanoparticle; a surrogate marker; and a means for detecting a specific chemical entity. In certain embodiments, nanoparticle-based assemblies include a payload for simultaneous diagnosis and treatment of disease. In further embodiments, a therapeutic drug and therapeutic drug marker are administered to a patient to monitor disease treatment. Bodily fluid samples are analyzed using sensor technology to detect the presence of surrogate and/or therapeutic drug markers to provide an efficient and accurate means for diagnosing a disease and/or monitoring disease treatment.

## Description

### Government Support

The subject matter of this application has been supported by a research grant from the National Science Foundation (Grant Number NSF: EEC 02-10580). Accordingly, the government may have certain rights in this invention.

### Cross-Reference to Related Applications

This application is a continuation-in-part of U.S. Application Serial No. 10/788,501, filed February 26, 2004; which is a continuation-in-part of U.S. Application Serial No. 10/178,877, filed June 24, 2002; which is a continuation-in-part of U.S. Application Serial No. 10/054,619, filed January 22, 2002. This application is also a continuation-in-part of U.S. Application Serial No. 10/744,789, filed December 23, 2003; which is a continuation-in-part of U.S. Application Serial No. 10/345,532, filed January 16, 2003. This application is also a continuation-in-part of U.S. Application Serial No. 10/274,829, filed October 21, 2002. This application is also a continuation-in-part of U.S. Application Serial No. 10/154,201, filed May 22, 2002, which claims the benefit of U.S. Application Serial No. 60/292,962, filed May 23, 2001. This application is also a continuation-in-part of U.S. Application Serial No. 10/722,620, filed November 26, 2003; which is a continuation of U.S. Application Serial No. 09/708,789, filed November 8, 2000, which claims the benefit of U.S. Application Serial No. 60/164,250, filed November 8, 1999, which is now abandoned. All of the afore-mentioned applications are hereby incorporated by reference herein in their entirety, including any figures, tables, or drawings.

### Field of the Invention

The present invention relates to non-invasive diagnosis, treatment, and monitoring of treatment, which includes non-invasive monitoring of target compound concentrations in bodily fluids; and more particularly, to a system and method for monitoring target compounds that are associated with a disease and/or therapeutic drug utilizing a breath detection system.

### Background of the Invention

There is a great need for the development of efficient and accurate systems for the detection, notification, and treatment of a variety of medical conditions, disorders, and diseases (hereinafter collectively referred to as "disease"). This requires an effective means for identifying in a patient the presence of specific chemical and/or biological agents associated with the disease such as nucleic acids, proteins, illicit drugs, toxins, pharmaceuticals, carcinogens, poisons, allergens, and infectious agents. Current methods of detecting such chemical or biological agents entail extraction of a sample (generally of blood) into organic solvents, followed by analysis using stand-alone analytical systems such as gas-liquid chromatography and/or mass spectroscopy. These methods are time-consuming and often expensive. Moreover, these methods do not include simultaneous treatment of the disease associated with the chemical or biological agent in the patient.

Once a disease is diagnosed, effective treatment for the disease using available drugs can be complicated due to individual clinical conditions. Historically, pharmaceutical compositions were delivered to patients according to standard doses based on the patient's weight. In the early 1970s, it was discovered with epileptic patients that pharmaceutical treatment with dosages adjusted according to blood concentration of the drug was far more efficient and demonstrated better seizure control and few side effects than with dosages adjusted according to patient weight.

It is now generally accepted that with many medications, it is necessary to monitor the concentration level of a drug in the blood stream in order to ensure optimal, therapeutic drug effect. Certain medications are ineffective if blood concentration levels are too low. Moreover, certain medications are toxic to the body when concentration levels in the blood are too high. It would also be valuable to have a means for monitoring drug concentration in blood for medications that do not require constant monitoring. By monitoring blood serum drug levels, medication dosage can be individualized within a therapeutically effective range.

For example, tricyclic or tetracyclic antidepressants (TCAs) require constant monitoring in patient blood. TCAs work by inhibiting serotonin and norepinephrine reuptake into the synaptic cleft. This group includes among its members the tricyclics imipramine, nortriptyline, and clomipramine, and the tetracyclics maprotiline and amoxapine. It is the inhibition of norepinephrine reuptake that is believed to cause TCAs side effects, which include sedation, manic episodes, profuse sweating, palpitations, increased blood pressure, tachycardia, twitches and tremors of the tongue or upper extremities, and weight gain. Compared with serotonin reuptake inhibitors (SSRIs) which are currently available, TCAs have very significant side effects, some virtually life threatening, and others merely difficult for patients to tolerate.

Although SSRIs are not more effective, and may actually be slightly less effective than some TCAs, TCAs are less attractive because they are more toxic than SSRIs and pose a greater threat of overdose. A TCA overdose results in central nervous system and cardiovascular toxicity making the relative risk of death by overdose with a TCA 2.5 to 8.5 times that with commercially available SSRI - Prozac. The greater danger with TCA is that side effects, as well as constant blood sampling, will persuade the patient not to continue treatment. Studies indicate that patients taking a classical antidepressant (TCA or MAOI) are three times as likely to drop out of treatment due to side effects and constant monitoring as patients taking Prozac.

Thus, therapeutically effective medications that require monitoring of blood serum drug levels are less likely to be prescribed by physicians in view of inconvenience in constant blood sampling and lack of patient compliance. Further, in the present era of cost-effective healthcare, considerations of prescription costs have become the primary issue for all aspects of laboratory operation. Individualization of drug therapy contributes to cost-effective patient management through detection and elimination of drug side effects; detection of unusual metabolism and adjustment of dosage based on individual metabolism; and detection of unusual metabolism and adjustment of dosage based on the effects on disease.

Drug level testing is especially important in patients being administered medications where the margin of safety between therapeutic effectiveness and toxicity is narrow. Drugs such as procainamide or digoxin, which are used to treat arrhythmia; dilantin or valproic acid, which are used to treat seizures; and gentamicin or amikacin, which are antibiotics used to treat infections, are examples of medications having a narrow margin of safety and therapeutic effectiveness with administration.

Currently available tests for therapeutic drug monitoring are invasive, difficult to administer, and/or require an extended period of time for analysis. Such tests are generally complex, requiring a laboratory to perform the analysis. Healthcare providers' offices rarely possess appropriate testing technology to analyze blood samples and must therefore send the samples to an off-site laboratory or refer the patient to the laboratory to have their blood drawn, which results in an extended time period for analysis. In the process of transfer to and from a laboratory, there is a greater likelihood that samples will be lost or mishandled, or that the incorrect results are provided to the healthcare provider, which could be detrimental to the patient's health and well-being. Further, those on-site test devices that are presently available for assessing drug concentration levels in blood are expensive. Reference laboratories using sophisticated techniques such as gas chromatography-mass spectrometry typically conduct complex and expensive toxicological analyses to determine the quantity of a medication.

It has been found that the concentration of drug in the blood may not directly reflect the concentrations at the cellular level, where most drugs exert their biological effects. The pharmacodynamics of a drug also exhibit wide inter- and intra-individual variation. The drug concentration at the site of action probably relates best with clinical responses; however, it is typically difficult or impossible to measure. Although plasma drug concentrations often provide an informative and feasible measurement for defining the pharmacodynamics of medications, they do not consistently provide an accurate report of drug disposition in a patient.

There are generally four processes by which drug disposition takes place: absorption, distribution, metabolism, and excretion. Absorption of a drug is generally dictated by route of drug administration *(i.e.,* intravenous (IV), intramuscular (IM), subcutaneous (SC), topical, inhalation, oral, rectal, sublingual, *etc.*); drug factors (*i.e.*, lipid solubility); as well as host factors *(i.e.,* gastric emptying time). Alterations in drug absorption may affect the therapeutic effectiveness of the drug.

Factors related to drug distribution include body fat, protein binding, and membranes. Because lipid soluble drugs tend to dissolve in fat, drugs can build up to very high, potentially toxic, levels in a patient with a high percentage of body fat. There are several drugs available that have a high affinity for serum proteins. Protein binding limits the therapeutic effectiveness of the drug. Membranes such as the blood brain barrier sometimes make it difficult for the drug to be properly distributed.

All tissues in the body can contribute to the metabolism of a drug. For example, the liver, kidney, lungs, skin, brain, and gut can all be involved in metabolizing a drug. Physiologically, metabolism can increase the activity, decrease the activity, or have no effect on the activity of a drug. Because metabolism of a drug differs from one patient to another, the dosage required for a drug can differ from patient to patient.

Routes of drug elimination include the kidney, liver, gastrointestinal tract, lungs, sweat, lacrimal fluid, and milk. All of these processes (absorption, distribution, metabolism, and excretion), which can occur at varying times after drug administration, affect the level of pharmacologically effective drug in a patient. Thus, current methods for analyzing a blood sample to assess plasma drug concentrations only provides a snapshot for defining the pharmacodynamics of a drug and does not consistently provide an accurate report of drug disposition in a patient.

Three recent advancements in medicine are particularly germane to expanding the potential of detecting chemical and/or biological agents, especially with regard to therapeutic drug monitoring as well as the diagnosis and treatment of disease: nanotechnology, biodetectors (biosensors), and the identification of biomarkers for specific diseases and/or therapeutic drugs. Nanotechnology, such as nanoparticles, offers many advantages when used for applications such as the delivery of bioactive agents *(i.e.,* DNA, AIDS drugs, gene therapy, immunosuppressants, chemotherapeutics), and drug uptake and degradation (i.e., enzyme encapsulation). For example, nanoparticles have been proposed as providing site-specific distribution of drugs to a target site. Appropriately sized particles have been proposed wherein such particles can be delivered to selected tissues to release their drug "payload" in a controlled and sustained manner.

Aptamers have recently been identified as potentially effective biosensors for molecules and compounds of scientific and commercial interest (see Brody, E.N. and L. Gold, "Aptamers as therapeutic and diagnostic agents," J. Biotechnol., 74(1):5-13 (2000) and Brody et al., "The use of aptamers in large arrays for molecular diagnostics," Mol. Diagn., 4(4):381-8 (1999)). Aptamers have demonstrated greater specificity and robustness than antibody-based diagnostic technologies. In contrast to antibodies, whose identification and production completely rest on animals and/or cultured cells, both the identification and production of aptamers takes place *in vitro* without any requirement for animals or cells.

Aptamer synthesis is potentially far cheaper and reproducible than antibody-based diagnostic tests. Aptamers are produced by solid phase chemical synthesis, an accurate and reproducible process with consistency among production batches. An aptamer can be produced in large quantities by polymerase chain reaction (PCR) and once the sequence is known, can be assembled from individual naturally occurring nucleotides and/or synthetic nucleotides. Aptamers are stable to long-term storage at room temperature, and, if denatured, aptamers can easily be renatured, a feature not shared by antibodies. Furthermore, aptamers have the potential to measure concentrations of ligand in orders of magnitude lower (parts per trillion or even quadrillion) than those antibody-based diagnostic tests. These inherent characteristics of aptamers make them attractive for diagnostic applications.

A number of "molecular beacons" (such as fluorescence compounds) can be attached to aptamers to provide a means for signaling the presence of and quantifying a target chemical or biological agent. For instance, an aptamer having such a signaling presence that is specific for cocaine has been synthesized (Stojanovic, M.N. et al., "Aptamer-based folding fluorescent sensor for cocaine," J. Am. Chem. Soc., 123(21):4928:31 (2001)). A fluorescence beacon, which quenches when cocaine is reversibly bound to the aptamer is used with a photodetector to quantify the concentration of cocaine present. Aptamer-based biosensors can be used repeatedly, in contrast to antibody-based tests that can be used only once.

Of particular interest as a beacon are amplifying fluorescent polymers (AFP). AFPs with a high specificity to TNT and DNT have been developed. It has been noted that a detector based on AFP technology, with high specificity to TNT and DNT, can also detect propofol, an intravenous anesthetic agent, in extremely low concentration. The combination of AFP and aptamer technologies holds the promise of robust, reusable biosensors that can detect compounds in minute concentrations with high specificity.

Medical science has also recognized the need to control, regulate, target, and monitor the release of drugs in the body. As noted above, mechanisms of drug metabolism are extremely complex and are influenced by a number of factors including competitive binding on protein and red blood cells with other molecules; enzymatic activity, particularly in the liver; protein, and red blood cell concentration; and a myriad of other factors. Thus, the goals have been to provide: 1) less frequent drug administration, 2) constant and continuous therapeutic levels of a drug in the systemic circulation or at a specific target organ site, 3) a reduction in undesirable drug side effects, and 4) a reduction in the amount and dose concentration required to realize the desired therapeutic benefit.

During the past decade, a wide variety of drug delivery systems have been designed and evaluated which include, for example, 1) drug carriers based on, for example, proteins, polysaccharides, synthetic polymers, erythrocytes, DNA, and liposomes/microspheres containing an entrapped drug. However, very little technology is available that can detect and notify the user of a specific medical state in real-time as well as allow convenient, simultaneous treatment and monitoring of treatment of the medical state.

It is therefore desirable to develop a system and method that accurately and efficiently detects/screens for target chemical compounds associated with a disease while simultaneously treating the corresponding disease and monitoring the treatment, which would provide a significant cost and time benefit, expand medical practice, as well as improve patient quality of life. There is also a need for a drug treatment and monitoring system capable of continuously monitoring drug concentration levels (to assess drug disposition) at remote locations and/or non-laboratory settings to monitor the therapeutic efficacy of the drug.

### Brief Summary of the Invention

The subject invention provides non-invasive systems and methods for real-time, simultaneous diagnosis and treatment of disease as well as real-time monitoring of treatment. In particular, the subject invention utilizes sensors that detect target compounds in fluid compositions (such as gaseous and liquid compositions), wherein target compounds are associated with either disease or therapeutic treatment.

For disease diagnosis, nanostructure-based assemblies that release a target compound (the target compound for disease diagnosis is hereinafter referred to as a "surrogate marker") are provided to a patient. The nanostructure-based assemblies of the invention are designed to release the surrogate marker in response to the presence of a specific chemical entity (SCE) or combination of SCEs in the patient's body, wherein the SCE is associated with a disease of interest. The surrogate marker is preferably detectable in bodily fluids (such as exhaled breath, urine, blood, *etc.*) to indicate that the SCE was present in the patient's body and enable diagnosis of the disease associated with the SCE.

In one embodiment, nanostructure-based assemblies of the invention are designed with the ability to differentiate between different types of blood cells. Surrogate markers are released from the nanostructure-based assemblies in the presence of specific types of blood cells to notify the presence and/or quantity of the specific blood type. With this information, the user can calculate the concentration of different blood cells in the patient.

In addition to providing notification/diagnosis, the systems and methods of the invention also enable substantially simultaneous treatment of the diagnosed disease. In one embodiment, nanostructure-based assemblies are provided to a patient wherein treatment for a specific disease is locally delivered upon detection of an SCE associated with the disease to allow for substantially simultaneous diagnosis and treatment.

In operation, a patient is administered a composition containing the nanostructure-based assemblies of the invention and thereafter, a sample of the patient's bodily fluid is collected and analyzed using a sensor of the invention. The results from the sensor are communicated to the user for use in real-time diagnosis of a medical disease. For real-time, simultaneous diagnosis and treatment, the nanostructure-based assembly of the invention further includes a means for treating the disease, which is released upon detection of an SCE associated with the disease.

In a preferred embodiment, the nanostructure-based assembly of the invention is composed of a nanoparticle that contains the following components: (a) a means for detecting an SCE; and (b) a surrogate marker. For simultaneous diagnosis and treatment of disease, the nanoparticle preferably contains an additional component, (c) a "payload," for treating the disease that is associated with the SCE. Any combination of these components can be attached to any surface of the nanoparticle.

Further embodiments of the invention enable real-time monitoring of disease treatment. Therapeutic drug concentration in blood is monitored by detecting, quantifying, and/or trending a target compound in exhaled breath (the target compound for therapeutic drug monitoring is hereinafter referred to as "therapeutic drug marker"), where the therapeutic drug marker is associated with a therapeutic drug. Sensors that analyze for exhaled breath components are preferably utilized to detect a therapeutic drug marker.

In related embodiments, a therapeutic drug is administered to a patient, either via nanostructure-based assemblies or using other routes of administration. After therapeutic drug administration, a therapeutic drug marker is presented in the patient that is detectable in exhaled breath by a sensor of the subject invention. In certain embodiments of the invention, the therapeutic drug marker is the therapeutic drug itself or a metabolite thereof, which is detectable in exhaled breath. In other embodiments, the therapeutic drug marker is a compound that is concurrently administered with the therapeutic drug to the patient.

As contemplated herein, the blood concentration of the therapeutic drug and the exhaled concentration of the therapeutic drug marker can be correlated. For example, in certain embodiments the blood concentration of the therapeutic drug and the exhaled concentration of the therapeutic drug marker are substantially proportional. By using a sensor of the subject invention to analyze the concentration of a therapeutic drug marker in exhaled breath, the present invention enables non-invasive, continuous monitoring of therapeutic drug concentration in blood.

One method of the subject invention includes the steps of administering at least one therapeutic drug to a patient to treat a disease and measuring thereafter the concentration of one or more therapeutic drug markers in the patient's exhaled breath. These measured markers can then be used to quantify the concentration of therapeutic drug(s) in the patient's blood as well as trend the delivered drug and ultimately determine the pharmacodynamics/pharmacokinetics of the drug.

The present invention can be used to diagnose, notify, and track the progress of a disease and/or therapeutic interventions for a wide variety of disease states in a convenient, non-invasive, real-time manner using a point-of-care (POC) approach. The subject invention can be used either in a clinical setting (such as a health care provider area) or patient-based location (such as a patient's home).

In certain embodiments, the systems and methods of the invention include a reporting system capable of tracking marker concentration (such as surrogate marker or therapeutic drug marker concentration) and providing necessary outputs, controls, and alerts. The tracking system can remotely or proximately track marker concentration.

Thus, the invention provides novel systems and methods for improving the quality of health care by enabling the following benefits in a non-invasive, real-time manner: 1) allow early detection of disease and identify those at risk of developing the disease, 2) provide an indication of the prognosis of the disease, 3) allow for accurate monitoring of therapeutic efficacy and drug compliance, 4) allow for detection of disease recurrence; and 5) allow for focused treatment of the disease, disorder, or condition.

### Brief Description of the Figures

**Figure 1** shows a capnogram of a single respiratory cycle and a capnogram of several breaths from a patient with obstructive lung disease.
**Figure 2** shows a gas sensor chip, which may be utilized as the sensor for the present invention.
**Figures 3A and 3B** illustrate embodiments of the invention.

### Detailed Description of the Invention

The present invention is directed to the efficient, accurate, and real-time identification, notification, and/or treatment of a disease as well as real-time point-of care monitoring of delivered treatment. The systems and methods of the invention apply sensors to a sample of bodily fluid to assess the presence and/or concentration of a target compound. Target compounds of the invention are preferably surrogate markers or therapeutic drug markers.

In certain embodiments, nanostructure-based assemblies are provided. Nanostructure-based assemblies include a nanoparticle, a means for detecting a target specific chemical entity (SCE) associated with a disease, and a surrogate marker detectable in bodily fluids. In related embodiments, nanostructure-based assemblies further include a payload to provide localized treatment for the identified disease.

To diagnose a disease of interest, nanostructure-based assemblies are administered to a patient. Thereafter, a bodily fluid sample is collected from the patient, to which sensor technology is applied to assess the presence of surrogate markers. A surrogate marker is released into the patient when a nanostructure-based assembly is in the presence of a target SCE. Specifically, bioactive interaction between an SCE-detector and a target SCE induces the release of the surrogate marker from the nanoparticle. Advantageously, the concentration of the released surrogate marker can be correlated to the amount of SCE present in the bodily fluid sample, which can be measured using quantitative sensor technology known in the art. In a preferred embodiment, the concentration of the released surrogate marker is proportional to the amount of SCE present in the bodily fluid sample.

In certain embodiments, substantially simultaneous and localized treatment is delivered with disease diagnosis. With these embodiments, the nanostructure-based assembly comprises a nanoparticle, a means for detecting an SCE, a surrogate marker, and a payload. In operation, these nanostructure-based assemblies are administered to a patient. Both the surrogate marker and payload are released into the patient when the nanostructure-based assemblies are in the presence of a target SCE. A bodily fluid sample is collected thereafter from the patient and sensor technology is applied to detect the presence of a surrogate marker. Detection and/or quantification of a surrogate marker in a bodily fluid sample enables the user to not only diagnose the disease associated with the SCE but also to verify delivery of treatment for the disease.

The present invention also provides systems and methods for non-invasive monitoring of therapeutic drug concentration in blood. Preferably, after administering a therapeutic drug to a patient to treat a disease of interest, the patient's exhaled breath is analyzed for the presence and/or quantity of therapeutic drug marker(s). Accordingly, the subject invention enables a user to provide a patient the maximum benefit from a therapeutic drug while minimizing risks for toxicity.

### Definitions

Unless otherwise stated, the following terms used in the specification and claims have the meanings given below.

The term "specific chemical entity" or "SCE" refers to a naturally occurring or synthetic compound in the body that has a particular molecular trait to make it useful for diagnosing a disease and for measuring or indicating the effects or progress of a disease. The term SCE also refers to, without limitation, any substance (such as an analyte, biomarker, and chemical and/or biological agents) that can be measured in an analytical procedure. Common specific chemical entities found in a person's bodily fluids (i.e., breath, blood, or urine), and the respective diagnostic disease associated with the SCE include, but are not limited to, acetaldehyde (source: ethanol; diagnosis: intoxication), acetone (source: acetoacetate; diagnosis: diet; ketogenic/diabetes), ammonia (source: deamination of amino acids; diagnosis: uremia and liver disease), CO (carbon monoxide) (source: CH₂Cl₂, elevated % COH; diagnosis: indoor air pollution), chloroform (source halogenated compounds), dichlorobenzene (source: halogenated compounds), diethylamine (source: choline; diagnosis: intestinal bacterial growth), H (hydrogen) (source: intestines; diagnosis: lactose intolerance), isoprene (source: fatty acid; diagnosis: metabolic stress), methanethiol (source: methionine; diagnosis: intestinal bacterial overgrowth), methylethylketone (source: fatty acid; diagnosis: indoor air pollution/diet), O-toluidine (source: carcinoma metabolite; diagnosis: bronchogenic carcinoma), pentane sulfides and sulfides (source: lipid peroxidation; diagnosis: myocardial infarction), H₂S (source: metabolism; diagnosis: periodontal disease/ovulation), MeS (source: metabolism; diagnosis: cirrhosis), and Me₂S (source: infection; diagnosis: trench mouth).

The term "biosensor" relates to the use of naturally occurring and/or synthetic compounds as highly specific and extraordinarily sensitive detectors of various types of SCEs of disease. In certain embodiments of the invention, naturally-occurring compounds such as antibodies are used to provide molecular recognition for a target SCE in diagnostic assays. Other embodiments of the invention use synthetic compounds manufactured to mimic naturally-occurring mechanisms of DNA, RNA, and protein synthesis in cells to facilitate the detection of a target SCE. Further embodiments of the invention use biosensors to recognize target compounds such as surrogate markers or therapeutic drug markers in bodily fluid samples.

The term "aptamer," as used herein, refers to a non-naturally occurring oligonucleotide chain that has a specific action on a target compound (such as a therapeutic drug marker, surrogate marker, or an SCE of interest). A specific action includes, but is not limited to, binding of the target compound, catalytically changing the target compound, and reacting with the target compound in a way that modifies/alters the target compound or the functional activity of the target compound. The aptamers of the invention preferably specifically bind to a target SCE and/or react with the target SCE in a way which modifies/alters the SCE or the functional activity of the SCE.

Aptamers include nucleic acids that are identified from a candidate mixture of nucleic acids. In a preferred embodiment, aptamers include nucleic acid sequences that are substantially homologous to the nucleic acid ligands isolated by the SELEX method. Substantially homologous is meant a degree of primary sequence homology in excess of 70%, most preferably in excess of 80%.

The "SELEX™" methodology, as used herein, involves the combination of selected nucleic acid li gands, which interact with a target SCE in a desired action, for example binding to a protein, with amplification of those selected nucleic acids. Optional iterative cycling of the selection/amplification steps allows selection of one or a small number of nucleic acids, which interact most strongly with the target SCE from a pool, which contains a very large number of nucleic acids. Cycling of the selection/amplification, procedure is continued until a selected goal is achieved. The SELEX methodology is described in the following U.S. patents and patent applications: U.S. patent application Serial No. 07/536,428 and U.S. patent Nos.: 5,475,096 and 5,270,163.

The term "indicator aptamers," as used herein, refers to aptamers to which molecular beacons are attached, such as those described in U.S. Patent Nos. 6,399,302 and 5,989,823.

The term "molecular beacons," as used herein, refers to a molecule or group of molecules (i.e., a nucleic acid molecule hybridized to an energy transfer complex or chromophore(s)) that can become detectable and can be attached to a biosensor under preselected conditions. For example, an embodiment of the present invention includes an aptamer-bound fluorescence beacon that (a) quenches when a target compound (such as a therapeutic or surrogate marker) or SCE is reversibly bound to the aptamer and (b) is detectable with a photodetector to quantify the concentration of a target compound or SCE that is present.

The term "disease," as used herein, encompasses a condition (*i.e.,* drug abuse), disease state (*i.e.,* infectious diseases), disorder *(i.e.,* neurological disorders), or a normal or pathologic process that occurs in a patient (i.e., drug metabolism), any one or combination of which is of interest to a healthcare provider.

The term "bodily fluid," as used herein, refers to a mixture of molecules obtained from a patient. Bodily fluids include, but are not limited to, exhaled breath, whole blood, blood plasma, urine, semen, saliva, lymph fluid, meningal fluid, amniotic fluid, glandular fluid, sputum, feces, sweat, mucous, and cerebrospinal fluid. Bodily fluid also includes experimentally separated fractions of all of the preceding solutions or mixtures containing homogenized solid material, such as feces, tissues, and biopsy samples.

The term "SCE-detector" or "SCE-detecting means," as used herein, refers to the use of biosensors, such as naturally-occurring and/or synthetic compounds, as highly specific and sensitive detectors of various types of SCEs. SCE-detectors of the invention can include naturally-occurring compounds such as antibodies, proteins, receptor ligands, and receptor proteins, any of which can provide molecular recognition for an SCE. Alternatively, the subject invention can use synthetic compounds such as aptamers, which can mimic naturally occurring mechanisms of DNA, RNA, and protein synthesis in cells, to facilitate SCE detection.

The term "surrogate marker" or "therapeutic drug marker," as used herein, refers to a molecule or compound that is innocuous to the patient and detectable by means of its physical or chemical properties. As such, surrogate and/or therapeutic drug markers of the invention are detectable by a number of sensor technologies known in the art including, but not limited to, flow cytometers, semiconductive gas sensors; mass spectrometers; infrared (IR), ultraviolet (UV), visible, or fluorescence spectrophotometers; gas chromatography, conductive polymer gas sensor technology; surface acoustic wave gas sensor technology; immunoassay technology, and amplifying fluorescent polymer (AFP) sensor technology. The surrogate or therapeutic drug markers of the invention include federally approved products categorized as GRAS ("generally recognized as safe") as well as other compounds not formally designated as GRAS which have suitable toxicological and physicochemical properties to be detected in accordance with the systems and methods of the subject invention. In preferred embodiments, the surrogate or therapeutic drug marker is a volatile marker detectable in bodily fluids, in particular blood and breath.

A "patient," as used herein, describes an organism, including mammals, to which treatment with the compositions according to the present invention is provided. Mammalian species that benefit from the disclosed methods of treatment include, but are not limited to, apes, chimpanzees, orangutans, humans, monkeys; and domesticated animals (e.g., pets) such as dogs, cats, mice, rats, guinea pigs, and hamsters.

As used herein, the term "pharmaceutically acceptable carrier" means a carrier that is useful in preparing a pharmaceutical composition that is generally compatible with the other ingredients of the composition, not deleterious to the patient, and neither biologically nor otherwise undesirable, and includes a carrier that is acceptable for veterinary use as well as human pharmaceutical use. "A pharmaceutically acceptable carrier" as used in the specification and claims includes both one and more than one such carrier.

As used herein, a "biodegradable" substance refers to a substance that can be decomposed by biological agents or by natural activity within an organism. Examples of contemplated biodegradable polymers include, but are not limited to: polyesters such as poly(caprolactone), poly(glycolic acid), poly(lactic acid), and polyhydroxybutrate; polyanhydrides such as poly(adipic anhydride) and poly(maleic anhydride); polydioxanone; polyamines; polyamides; polyurethanes; polyesteramides; polyorthoesters; polyacetals; polyketals; polycarbonates; polyorthocarbonates; polyphosphazenes; poly(malic acid); poly(amino acids); polyvinylpyrrolidone; poly(methyl vinyl ether); poly(alkylene oxalate); poly(alkylene succinate); polyhydroxycellulose; chitin; chitosan; and copolymers and mixtures thereof.

As used herein, a "biocompatible" substance includes those substances that are compatible with and have demonstrated no significant toxic effects on living organisms. Examples of contemplated biocompatible polymers include PLG (Poly(lactide-co-glycolide)), poly(ethylene glycol), and copolymers of poly(ethylene oxide) with poly(L-Lactic acid) or with poly(β-benzyl-L-aspartate). In a preferred embodiment, biocompatibility includes immunogenic compatability. An immunogenically compatible substance can include a substance that, when introduced into a body, does not significantly elicit humoral or cell-based immunity.

As used herein, "treating" or "treatment" includes: (1) preventing the disease (i.e., inhibiting the development of clinical symptoms of a disease in a mammal that may be exposed to or predisposed to the disease but does not yet experience or display symptoms of the disease); (2) inhibiting the disease (i.e., arresting the development of the condition or its clinical symptoms), or (3) relieving the disease (i.e., causing regression of the disease or its clinical symptoms).

The term, "payload" or "payload material," as used herein, refers to a therapeutic drug for treatment of a disease. Preferably, the payload of the invention is a therapeutic drug that treats a disease associated with an SCE.

As used herein, the term "therapeutic drug" or "drug" refers to a substance used in the diagnosis and/or treatment of a disease. In certain embodiments, the concentration of the therapeutic drug in a patient's blood stream is monitored via exhaled breath analysis to ensure a therapeutic drug level is within a clinically effective range. In other embodiments, the concentration of the therapeutic drug in a patient's blood stream is monitored via exhaled breath analysis to assess individual patient pharmacodynamics and/or pharmacokinetics of the drug.

The term "therapeutically effective amount," as used herein, means the amount of a compound that, when administered to a mammal for treating a disease, is sufficient to effect such treatment for the disease. The "therapeutically effective amount" will vary depending on the medication, the disease state being treated, the severity of the disease treated, the age and relative health of the patient, the route and form of administration, the judgment of the attending medical practitioner, and other factors.

The term "pharmacodynamics," as used herein, refers to the interaction (biochemical and physiological) of a therapeutic drug with constituents of a patient body as well as the mechanisms of drug action on the patient body *(i.e.,* drug effect on body).

As used herein, the term "pharmacokinetics" refers to the mathematical characterization of interactions between normal physiological processes and a therapeutic drug over time (i.e., body effect on drug). Certain physiological processes (absorption, distribution, metabolism, and elimination) will affect the ability of a drug to provide a desired therapeutic effect in a patient. Knowledge of a drug's pharmacokinetics aids in interpreting drug blood stream concentration and is useful in determining pharmacologically effective drug dosages.

"Concurrent" administration, as used herein, refers to the administration of a marker suitable for use with the systems and methods of the invention (such as a surrogate marker or a therapeutic drug marker) to accomplish any of the objectives of the invention (such as disease diagnosis or monitoring therapeutic drug levels in blood stream). By way of example, a therapeutic drug marker can be provided in admixture with a therapeutic drug, such as in a pharmaceutical composition; or the marker and therapeutic drug can be administered to a patient as separate compounds, such as, for example, separate pharmaceutical compositions administered consecutively, simultaneously, or at different times. Preferably, if the therapeutic drug marker and the therapeutic drug are administered separately, they are administered within sufficient time from each other so that the concentration of the marker in exhaled breath is an accurate indicator of the concentration of therapeutic drug in the blood stream.

### Nanoparticles

Nanostructure-based assemblies offer timely, and effective detection, notification, and treatment of a disease of interest. Such assemblies are based on nanoparticles, which provide a mechanism for the targeted delivery and release of detectable markers and/or bioactive treatment agents to selected sites within the body.

According to the present invention, nanoparticles can be produced in a wide range of sizes and shapes, and composed of a wide range of materials, or combination of materials, optimized for in-vivo administration. Contemplated shapes include, but are not limited to, spherical, elliptical, cubic, cylindrical, tetrahedron, polyhedral, irregular- prismatic, icosahedral, and cubo-octahedral forms. Nanoparticles intended for *in-vivo* use are of any dimension, preferably with a maximum dimension less than 500 nm, so as to ensure proper distribution at the microvasculatoure level, without any occlusion of blood flow. More preferably, the nanoparticles of the subject invention are of a maximum dimension less than 100-150 nm. The "maximum dimension" of a nanoparticles is the maximum distance between any two points in the nanoparticle. In a preferred embodiment, the nanoparticles are in the form of tubular bodies (also known as "nanotubes"), which are either hollow or solid and include either open ends or one or both closed ends.

In accordance with the present invention, the nanoparticle-based assemblies are composed of biodegradable substances. In other embodiments, the nanoparticle-based assemblies of the invention are composed of biocompatibL e substances.

Methods of preparation of nanoparticles are well known in the art. For example, the preparation of monodisperse sol-gel silica nanospheres using the well-known Stober process is described in Vacassy, R. et al., "Synthesis of Microporous Silica Spheres," J. Colloids and Interface Science, 227, 302 (2000).

Nanoparticles, in accordance with the present invention, can be prepared from a single material or a combination of materials. For example, nanotubes can be prepared from either one or a combination of materials including, but not limited to, polymers, semiconductors, carbons, or Li⁺ intercalation materials. Metal nanoparticles include those made from gold or silver. Semi-conductor nanoparticles include those made from silicon or germanium. Polymer nanoparticles include those made from biocompatible or biodegradable polymers. The ability to make nanoparticles from a wide variety of materials or combination of materials allows the creation of nanoparticles with desired biochemical properties such as biocompatibility, including immunogenic compatibility, and/or, biodegradability. In comparison, certain biological delivery systems, such as viral vectors, can cause significant immunogenic phenomena.

Nanoparticles of the present invention can be synthesized using a template synthesis method. For example, nanoparticles can be synthesized using templates prepared from glass (Tonucci, R.J. et al., Science 258, 783 (1992)), xeolite (Beck, J.S. et al., J. Am. Chem. Soc., 114, 10834 (1992)), and a variety of other materials (Ozin, G.A., Adv. Mater., 4, 612 1992)). Alternatively, nanoparticles can be prepared using a self-assembly process, as described in Wang, Z.L., "Structural Analysis of Self-Assembling Nanocrystal Superlattices," Adv. Mater., 10(1):13-30 (1998).

In one embodiment, a nanostructure-based assembly of the invention contains a nanoparticle, which has one or more surfaces functionalized to allow attachment of SCE-detectors to the surface. Such "functionalized" nanoparticles have at least one surface modified to allow for directed (also referred to as "vectoring") delivery and/or controlled release of the surrogate marker (and payload, when available). In certain embodiments, the nanoparticle is formed with an interior void. Different chemical and/or biochemical functional groups can be applied to the inside and/or outside surfaces of the nanoparticle to enable the attachment of an SCE-detector, surrogate marker, and/or payload on a nanoparticle surface.

In another embodiment, the nanostructure-based assembly contains a nanoparticle formed with an interior void to contain a surrogate marker, a payload, and a detachable end-cap with an SCE-detector attached thereto. In the presence of a target SCE, the SCE-detector mechanically detaches the end-cap from the nanoparticle to release the surrogate marker for analysis by sensor technology. Simultaneously, the payload is released for the treatment of a disease.

In a preferred embodiment, the nanoparticle is in the form of a nanotube that is hollow and has a first open end and a second closed end. A surrogate marker and payload are enclosed within the hollow interior of the nanotube. The first open end is blocked with an aptamer-bound end-cap that prevents the unintentional release of the surrogate marker (and payload, when available) located within the hollow interior of the nanotube. Once in the presence of a target SCE, the aptamer binds with the SCE and causes the end-cap to dissociate from the nanotube and release the surrogate marker (and payload, when present).

Upon detecting a target SCE by the aptamer attached to the end-cap, the surrogate marker and payload are released with the uncapping of the nanoparticle. The uncapping mechanism may require the use of energy-bearing biomolecular motors such as, but not limited to, the actin-based system (Dickinson, R.B. and D.L. Purich, "Clamped filament elongation model for actin-based motors," Biophys J., 82:605-617 (2002)). Once the nanoparticle is uncapped, the released surrogate marker can then be detected using sensor technology known in the art including, but not limited to, gas chromatography, electronic noses, spectrophotometers to detect the surrogate marker's infrared (IF), ultraviolet (UV), or visible absorbance or fluorescence, or mass spectrometers. Further, the release of the payload ensures localized release of treatment at the desired organ or tissue site, thereby permitting enhanced, desired therapeutic activity and decreased use of dosage amounts.

### Nanotubes

A number of patents and publications describe nanoparticles in the form of tubes (nanotubes). For example, U.S. Patent No. 5,482,601 to Ohshima et al*.* describes a method for producing carbon nanotubes. Other methods for making and using nanotubes include the non-carbon nanotubes of Zettl et al., U.S. Patent No. 6,063,243, and the functionalized nanotubes of Fisher et al., U.S. Patent No. 6,203,814.

For nanotubes, synthesis occurs within the membrane pores of a microporous membrane or other solid, as described in Charles R. Martin, "Nanomaterials: A Membrane-Based Synthetic Approach," Science, 266:1961-1966 (1994), using electrochemical or chemical methods. Depending on the membrane and synthetic method used, the nanotubes may be solid or hollow. Template membrane pore diameters can be varied to produce nanotubes having diameters as small as 5 nm to as large as 100 µm. Likewise, the template membrane thickness can be varied to give nanotubes having a length from as small as 5 nm to as large as 100 µm. Preferably, when the nanotube is intended for *in vivo* use, the nanotube is of length less than 500 µm and diameter less than 200 nm. Especially preferred nanotubes for *in vivo* use have a maximum dimension less than 100 nm.

"Track-etch" polymeric or porous alumina membranes can be used in the preparation of nanotubes. Track-etch membranes prepared from polycarbonate and polyester are available from suppliers such as Osmonics (Minnetonka, MN) and Whatman (Maidstone, Kent UK). Track-etch membranes contain randomly distributed cylindrical pores of uniform diameter that run through the entire thickness of the membrane. Pore diameters as small as 10 nm are commercially available at pore densities of up to 10⁹ pores per square centimeter.

Porous alumina membranes, which are commercially available from Whatman (Maidstone, Kent UK), are prepared electronically from aluminum metal. Pore diameters as small as 5 nm can be achieved at pore densitites as high as 10¹¹ pores per square centimeter. Membranes can be prepared having the membrane thickness from as small as 100 nm to as large as 100 µm.

Nanotubes can be synthesized such that both ends of the nanotube are open. Alternatively, nanotubes having one or two open end(s) can be synthesized. Solid nanotubes can also be synthesized.

Nanotubes with one closed end can be produced by template synthesis, as described above. For example, nanotubes having one closed end can be prepared by terminating the pores in the alumina template into a non-porous alumina barrier layer prior to removal of the alumina template membrane from the substrate aluminum surface (Hornyak, G.L., et al., "Fabrication, Characterization and Optical Properties of Gold-Nanoparticle/Porous-Alumina Composites: The Non-Scattering Maxwell-Garnett Limit," J. Phys. Chem. B., 101:1548-1555 (1997)). Generally, the non-porous alumina barrier layer is removed when the alumina membrane is stripped off of the aluminum surface. However, if the template synthesis is completed before removal of the alumina from the aluminum, the bottoms of the nanotubes are closed. Dissolution of the alumina then liberates the nanotubes that are closed at one end and open at the other end.

Suitable end-caps used to block a nanotube opening include, for example, nanoparticles having a diameter slightly larger than the inside diameter of the nanoparticle so as to occlude the open end of the nanoparticle. End-caps are any piece of matter and can be composed of materials that are chemically or physically similar (or dissimilar) to the nanoparticle. The end-cap can be a particle that has a maximum dimension of less than 100 µm. In a preferred embodiment, the end-cap is of a spherical or spheroidal form. However, end-caps of other shapes, including ellipsoidal, cylindrical, and irregular, can also be used.

A suitable end-cap can be attached to a nanotube by covalent bonds. For example, silica nanotubes and particles can be linked by disulphide bonds. Initially, the surface at the ends of silica nanotubes is functionalized with a -SH linker. This can be performed while the nanotubes are still embedded in the pores of the template membrane. This allows activation of the end surface without changing the chemical properties of the outer surface of the nanotubes.

If necessary, the inner surfaces of the nanotubes are protected with, for example, a silane group such as (Me-O)₃-(CH₂)₃-OH. After the protection step, the silica surface layers at the nanotube mouths are removed to expose fresh silica. The freshly-exposed silica will be reacted with the silane, such as (Me-O)₃-Si-(CH₂)₃-SH to attach the requisite -SH linker to the mouths of the nanotubes. The length of the alkyl chain in this silane can be varied to allow placement of the -SH linker any desired distance from the nanotube mouth. These -SH functionalities are then reacted with pyridine disulfide in order to obtain nanotubes with an activated disulfide bond at the nanotube ends.

The surface of the end-cap is then functionalized with the same -SH containing silane used on the mouths of the nanotubes. Hence, nanotubes with an activated disulfide at their mouths and end-caps with an -SH group on their surface are available for linkage through disulfide bond formation.

Other types of covalent bonds, for example amide and ester bonds, can be used to attach an end-cap to the nanotube. Siloxane based linking can also be used. This would be particularly useful when the cap is composed of silica as the silanol sites on the silica surface reacts spontaneously with siloxanes to form a covalent oxygen-silicon bond. For metal based nanotubes or end-caps, thiol linkers can be used for attachment. For example, molecule (Me-O)₃-Si-(CH₂)₃-SH could be attached to a silica nanotube and a gold nanoparticle attached as the end-cap using the -SH end of this molecule. It is well known that such thiols form spontaneous As-S bonds with gold surfaces.

Contemplated end-caps for the invention include nanoparticles that can be electrophoretically placed within the mouths of nanotubes so that the entire mouth of the nanotube is blocked when disulfide bonds are formed between the nanotube and the nanoparticle as described in Miller, S.A. and C.R. Martin, "Electroosmotic Flow in Carbon Nanotube Membranes," J. Am. Chem. Soc., 123(49):12335-12342 (2001).

For example, a nanotube containing membrane is mounted in a U-tube cell with Platinum electrodes immersed into the buffer solution on either side of the membrane. The -SH-functionalized end-caps are added to the cathode half-cell. The buffer solution is maintained at pH = 7 so that a small fraction of the -SH groups on the end-caps are deprotonated. These negatively charged particles are driven into the mouths of the nanotubes electrophoretically by using the Platinum electrodes to pass a constant current through the membrane. Hence, the electrophoretic force causes the end-caps to nestle into the nanotube mouths, where disulfide bond formation will occur.

As an alternative to the electrophoretic assembly method, -SH labeled end-caps can be suspended in solution together with the activated disulfide labeled nanotubes. Here, the nanoparticle caps can spontaneously self-assemble to the nanotubes. The self-assembly of gold nanospheres and latex particles to template prepared polymeric and metal nanowires is described by Sapp, S.A. et al., "Using Template-Synthesized Micro- and Nanowires as Building Blocks for Self-Assembly of Supramolecular Architectures," Chem. Mater., 11:1183-1185 (1999).

In addition to -SH linking, other covalent linking methods can be used to link nanotubes and end-caps. Non-covalent linking methods can be used. These include, for example, DNA hybridization (Mirkin, C.A., "Programming the Self-Assembly of Two and Three-Dimensional Architectures with DNA and Nanoscale Inorganic Building Blocks," Inorg. Chem., 39:2255-2272 (2000)), the biotin/avidin interaction (Connolly, S. and D. Fitzmaurice, "Programmed Assembly of Gold Nanocrystals in Aqueous Solution," Adv. Mater., 11:1202-1205 (1999)), and antigen/antibody interactions (Shenton, W. et al., "Directed Self-Assembly of Nanoparticles into Macroscopic Materials Using Antibody-Antigen Recognition," Adv. Mater., 11:449 (1999)).

Preferred nanotubes are those comprising silica or polymers. Silica nanotubes can be prepared using sol-gel template synthesis, as described in Lakshmi, B.B. et al., "Sol-Gel Template Synthesis of Semiconductor Oxide Micro- and Nanostructures," Chem. Mater., 9:2544-2550 (1997); Lakshmi, B.B. et al., "Sol-Gel Template Synthesis of Semiconductor Nanostructures," Chem Mater., 9:857-862 (1997). The template membrane is immersed into a standard tetraethylorthosilicate sol so that the sol fills the pores. After the desired emersion time, the membrane is removed, dried in air, and then cured at 150°C. This yields silica nanotubes lining the pore walls of the membrane plus silica surface films on both faces of the membrane. The surface films are removed by briefly polishing with slurry of alumina particles. The nanotubes are then liberated by dissolving the template membrane and collected by filtration.

The outside diameter of the nanotube can be controlled by varying the pore diameter of the template membrane, the length of the nanotube can be controlled by varying the thickness of the template membranes, and the inside diameter of the nanotube can be controlled by varying the immersion time in the sol.

Polymer nanotubes can be prepared from many substances that are composed of monomer units. "Monomer units," as used herein, refers to the individual moieties that are repeated to form "polymers." Multiple monomer units are covalently attached when tin the form of a backbone of a polymer. Polymers that are made from at least two different types of monomer units are referred to as "copolymers." Polymerizing or copolymerizing describes the process by which multiple monomers are reacted to form covalently linked monomer units that form polymers or copolymers, respectively. A discussion of polymers, monomer units, and the monomers from which they are made may be found in Stevens, Polymer Chemistry: An Invitation, 3rd ed., Oxford University Press (1999).

Polymeric nanotubes can be prepared using a solution deposition method as described in Depak, V.M. and C.R. Martin, "Preparation of Polymeric Micro- and Nanostructures Using a Template-Based Deposition Method," Chem. Mater., 11:1363-1367 (1999).. This method entails depositing a solution of the desired polymer within the pores of the template membrane and allowing the solvent to evaporate. In addition, polymer nanotubes can be prepared by polymerizing a monomer of a monomer within the pore as described by Martin, C.R., "Template Synthesis of Electronically Conductive Polymer Nanostructures," Acc. Chem. Res., 28:61-68 (1995).

Preferred polymers include polystyrene, polyorganosiloxane, poly(methyl methacrylate), polystyrene, polylactic acids, and other biodegradable polymers, acrylic latexes, polyorganosiloxane, cellulose, polyethylene, poly(vinyl chloride), poly(ethyl methacrylate), poly(tetrafluoroethylene), poly(4-iodostyrene/ divinylbenzene), poly(4-vinylpyridine/divinylbenzene), poly(styrene/ divinyl benzene), crosslinked melamine particles, phenolic polymer colloids, polyamide 6/6, natural rubber, naturally occurring biopolymers such as algenates, and collagen, or mixtures thereof.

When the nanotubes are to be introduced into a patient, for example, when used as a nanostructure-based assembly for the detection, notification, and treatment of a disease, biodegradable polymers and biocompatible polymers are especially preferred. Examples of useful biodegradable polymers include polyesters, such as poly(caprolactone), poly(glycolic acid), poly(lactic acid), and poly(hydroxybutryate); polyanhydrides, such as poly(adipic anhydride) and poly(maleic anhydride); polydioxanone; polyamines; polyamides; polyurethanes; polyesteramides; polyorthoesters; polyacetals; polyketals; polycarbonates; polyorthocarbonates; polyphosphazenes; poly(malic acid); poly(amino acids); polyvinylpyrrolidone; poly(methyl vinyl ether); poly(alkylene oxalate); poly(alkylene succinate); polyhydroxycellulose; chitin; chitosan; and copolymers and mixtures thereof.

Preferably, biocompatible are characterized by immunogenic compatibility. Examples of biocompatible polymers that can be used in the manufacture of nanotubes of the invention include PLG [Poly(lactide-co-glycolide)], poly(ethylene glycol), copolymers of poly(ethylene oxide) with poly(L-Lactic acid) or with poly(β-benzyl-L-aspartate. In addition, a number of approaches can be used to make a nanotube surface biocompatible and "stealthy." For example, this can be accomplished by attaching a PEG-maleimide to the chain-end thiols on the outer surfaces of the nanotube. If the nanotube is composed of Au or similar metals, the PEG chain can be attached by a thiol linker as described in Yu, S.; Lee, S.B.: Kang, M.: Martin, C.R. "Size-Based Protein Separations in Poly(ethylene glycol)-Derivatized Gold Nanotubule Membranes," Nano Letters, 1:495-498 (2001). Other examples of biocompatible polymers and surface treatments can be found in Majeti N.V. Ravi Kumar, "Nano and Microparticles as Controlled Drug Delivery Devices" J. Pharm. Pharmaceut. Sci. 3(2): 234-258 (2000), the contents of which are incorporated by this reference.

In one embodiment of the invention, a nanostructure-based assembly includes a nanotube with a hollow interior comprising a surrogate marker and/or payload material. The nanotube is constructed using known methods such as those disclosed in U.S. Patent Application Serial No. 10/274,829, filed October 21, 2002. The nanotube further includes an SCE detecting means for localizing the nanostructure-based assembly to a target SCE. The surrogate marker (and payload, when present) material are released from the nanostructure-based assembly when in the presence of a target SCE.

In a related embodiment, release of the surrogate marker and/or payload material in the hollow void is achieved by "uncapping" the nanotube. An end-cap is placed over an opening to the void to function as a means for controlling the release of the contents therein (i.e., surrogate marker and/or payload material). Methods for attaching an end-cap to a nanoparticle include, but are not limited to, using: electrostatic attraction, hydrogen bonding, acid and/or basic sites located on the end-cap/nanoparticle, covalent bonds, and other chemical linkages.

In a preferred embodiment, the detecting means is attached to the end-cap to affect the release of the surrogate marker and/or payload material via uncapping of the nanoparticle. For example, the uncapping mechanism is based upon the detection by the detecting means of certain SCEs including for example, surface markers on cell types *(i.e.,* cancer cells), proteins in the blood (*i.e.,* PSA for prostate cancer), or drugs in the body (i.e., illicit drugs or therapeutic drugs). The uncapping mechanism may require the use of energy-bearing biomolecular motors such as, but not limited to, the actin-based system (Dickinson, R.B. and D.L. Purich, "Clamped filament elongation model for actin-based motors," Biophys J., 82:605-617 (2002)).

The released surrogate marker can then be detected using sensor technology known in the art including, but not limited to, gas chromatography, electronic noses, spectrophotometers to detect the detectable marker's infrared (IF), ultraviolet (UV), or visible absorbance or fluorescence, or mass spectrometers.

### Functionalization of the Nanoparticles

According to the present invention, nanoparticles can be prepared having different chemically or biochemically functionalized surfaces to enable attachment of an SCE-detecting means, surrogate marker, and/or payload. Methods used to functionalize a nanoparticle surface depend on the composition of the nanoparticle and are well known in the art. For example, functionalization of silica nanoparticles is accomplished using silane chemistry. With silane chemistry, different functional groups can be attached to the surfaces of the nanoparticle by attaching a functional group to the nanoparticle surface while the nanoparticles are embedded within the pores of the template. Then, a hydrolytically unstable silane is reacted with the surface silanol sites on the nanoparticle to obtain covalent oxygen/silicon bonds between the surface and the silane. Additional functional groups can also be attached to the nanoparticle surface after dissolution of the template.

The surface of polymer nanoparticles can also be functionalized using well known chemical methods. For example, methods employed for polylactide synthesis allow for differential end-functionalization. Polymerization occurs by an insertion mechanism mediated by Lewis acids such as Sn²⁺ whose bonds with oxygen have significant covalent character. An alcohol complexed with the metal ion initiates polymerization, which continues by stepwise ring-opening of the lactide monomers to generate a new alkoxide-metal complex capable of chain growth. The polymer molecular weight can be controlled by the molar ratio of initiating alcohol to the lactide monomer. The resulting polyester possesses directionality with a hydroxyl terminus (from the first monomer) and a functional group at the ester terminus determined by the structure of the initiating alcohol. The latter can contain a variety of functional groups to enable attachment of a detecting means, surrogate marker, and/or payload to a nanoparticle surface.

Alternatively, functional groups can be introduced by copolymerization. Natural amino acids are sterically similar to lactic acid but offer a variety of functional groups on their side chains (-OH, -CO₂H, -NH₂, -SH, *etc.).* Moreover, amino acids are found in all cell types, so that the polymer degradation products are non-toxic. Monomers derived from an amino acid and lactic acid can be synthesized by standard methods and used for random copolymerization with lactide. In accordance with the present invention, nanoparticles can have functional groups on any surface to enable the attachment of an SCE-detecting means, a surrogate marker, and/or a payload. Such functional groups allow the nanostructure-based assembly to be bioengineered to accomplish specific functions, such as detect, provide notification of, and treat specific diseases.

In addition, the detecting means, surrogate marker, and/or payload can be incorporated into the nanoparticle framework, which can include chitosan, PEGylated PLGA (poly(lactic-co-glycolic acid), or other PEGylated compounds. For example, a commercially available PEG-maleimide can be incorporated into chain-end thiols on the outer surface of the nanoparticles. Alternatively, the detecting means, surrogate marker, and/or paylo ad can be incorporated into nanoparticle frameworks composed of biodegradable and/or resorbable materials including, for example, polylactide based polymers as described above.

For nanoparticles comprising a hollow void in which the surrogate marker can be contained, a surro gate marker can be loaded into the void using an electrophoretic force. (See Miller, S.A. and C.R. Martin, "Electroosmotic Flow in Carbon Nanotube Membranes," J. Am. Chem. Soc., 123(49):12335-12342 (2001)). Alternatively, nanoparticles embedded within the synthesis membrane can be filled with a surrogate marker by vacuum filtering a solution containing the surrogate marker through the synthesis membrane. (See Parthasarathy, R. and C.R. Martin, Nature, 369:298 (1994)). For nanoparticles prepared by formation within an alumina template film prior to removal of the alumina from the underlying aluminum surface, they can be filled by simply applying a solution containing the surrogate marker to the surface of the film (where the opening to the hollow void is located) and allowing the solvent to evaporate. Multiple applications can be used, if needed.

In one embodiment, a detecting means is attached to the outer surface of the nanoparticle via any of the aforementioned functional groups. The controlled release of the surrogate marker and, when present, payload are accomplished by the release of the end-cap, which is attached to the nanoparticle via chemically labile bonds.

Yet another embodiment provides a nanoparticle that has the detecting means, the surrogate marker, and the payload (when present) applied to the outside, exposed surface of the nanoparticle. All of these components are attached to the surface of the nanoparticle via chemically labile bonds, which allow for the release of these components under specific conditions (such as release of a surrogate marker after SCE detector identification of an SCE).

In a related embodiment, aptamers are used as SCE detectors. Aptamers can be attached to proteins utilizing methods well known in the art (see Brody, E.N. and L. Gold, "Aptamers as therapeutic and diagnostic agents," J Biotechnol, 74(1):5-13 (2000) and Brody, E.N. et al., "The use of aptamers in large arrays for molecular diagnostics," Mol Diagn, 4(4):381-8 (1999)). For example, photo-cross-linkable aptamers allow for the covalent attachment of aptamers to proteins. Such aptamer-linked proteins can then be immobilized on a functionalized surface of a nanoparticle.

For example, aptamer-linked proteins can be attached covalently to a nanoparticle end-cap or to an exterior nanoparticle surface, including attachment of the aptamer-linked protein by functionalization of the surface. Alternatively, aptamer-linked proteins can be covalently attached to a nanoparticle surface via linker molecules. Non-covalent linkage provides another method for introducing aptamer-linked proteins to a nanoparticle surface. For example, an aptamer-linked protein may be attached to an nanoparticle surface by absorption via hydrophilic binding or Van der Waals forces, hydrogen bonding, acid/base interactions, and electrostatic forces.

An identification of an SCE by the SCE-detecting means affects the release of the surrogate marker from the nanoparticle. Because the surrogate marker is released from the nanoparticle only in the presence of an SCE, detection of the surrogate marker in a bodily fluid sample provides notice that the SCE is present in the patient and consequently, allows diagnosis of the specific disease associated with the SCE.

Further, the detection of an SCE can also cause the substantially simultaneous release of a payload, when provided, with the surrogate marker. The payload is designed to prevent, alleviate, and/or cure the specific disease associated with the SCE. Thus, with concentrated delivery of the payload agent at the desired organ or tissue site, specific therapeutic effects can now be realized with minimized side effects, thereby permitting enhanced desired therapeutic activity and the use of decreased dosage amounts. Thus, in those embodiments in which a payload is included in the nanostructure-based assembly, the detection of the surrogate marker in a bodily fluid sample would also serve as an indication that the payload has been released.

In certain embodiments, the nanostructure-based assemblies of the invention have the ability to differentiate between and provide a signal regarding different types of blood cells and their concentration in the patient. For example, levels of red blood cells (RBCs), white blood cells (WBCs), and platelets can be assessed using the systems and methods of the invention to diagnose and/or treat hematopoiesis abnormalities such as leukemia or assess changes in cellular content (for example, RBC content). Accordingly, the subject invention is useful in diagnosing and/or treating blood-based diseases or disorders including, without limitation, hemorrhagic diathesis (i.e., hemophilia, von Willebrand disease, Alexander's disease, Telfer's disease, Owren's parahemophilia, prothrombin deficiency); non-hemorrhagiparous coagulopathies (i.e., Fletcher factor deficiency, Flaujeac factor deficiency); thrombophilic coagulopathies (i.e., Ratnoff's disease, thrombomodulin deficiency); thrombocytopenia; anemias; and alterations in white blood cells (i.e., Pelger-Huët anomaly (PHA); Chediak-Higashi syndrome (CHS); Hegglin-May anomaly (HMA)).

### Specific Chemical Entities (SCEs)

An SCE can be 1) attached to different types of cells (i.e., surface markers of diseased or normal cells), or 2) located in various bodily fluids (i.e., circulating markers of inflammatory disorders or cancer; therapeutic or illicit drugs) such as the blood. Thus, the SCE can include, but is not be limited to, a biomarker or analyte such as a protein, DNA, RNA, oligonucleotides, sugars, nucleosides, nucleotides, antibodies or a variety of small therapeutic and/or illicit drug molecule targets.

Many types of important antigens on cell surfaces indicate the presence of a wide variety of disease states, ranging from cancer, inflammatory disorders, and infections to cardiovascular disease. Surface cell markers can help identify a diseased cell (i.e., malignancy) in two ways: 1) by being uniquely expressed (not ordinarily present on the surface in normal cells), or 2) by being expressed in a greatly altered density (i.e., marked overexpression of a surface cell marker). For example, in the case of blood malignancies such as lymphomas and leukemias, unique markers and clusters of surface markers can be used to accurately identify blood cancers. Accordingly, SCEs of the present invention can include, without limitation, surface markers that identify disease states, including those surface markers known to identify leukemias and lymphomas via immunophenotyping.

Examples of SCEs of the invention include, but are not limited to, (1) T cell markers (CD2, CD3, CD4, CD5, CD7, and CD8); B cell markers (CD19 and CD20); myeloid/monocytic markers (CD13, CD14, CD15, and CD33); maturity status markers (CD34, HLA-DR, and CD10 = CALLA) that form an acute leukemia surface antigen profile; (2) pan-T cell markers: CD2, CD3, CD5; CD4 (helper) and CD8 (suppressor); pan-B markers CD19 and CD20; CD5 and CD20 (co-expression frequently indicates neoplastic proliferations) that form a chronic lymphocytic leukemia (CLL) and lymphoma Profile; (3) hairy cell markers CD11c (complement receptor), CD 25 (IL-2 receptor), CD103, prolymphocytic/hairy cell marker FMC-7; B-lymphoid marker CD23 (evaluated in relationship to CD5 expression for the different diagnosis of CLL vs. MCL) that aid in diagnosing Hairy Cell Leukemia (HCL), Prolymphocytic Leukemia (PLL), or Mantle Cell Lymphoma / Leukemia; and (4) CD1, CD15, and CD30 (Ki-1) that indicate anaplastic lymphoma and Hodgkin's Disease.

Additional SCEs of the invention include, but are not limited to, metabolites or compounds commonly found in bodily fluids including, but not limited to, acetaldehyde (source: ethanol; diagnosis: intoxication), acetone (source: acetoacetate; diagnosis: diet or ketogenic/diabetes), ammonia (source: deamination of amino acids; diagnosis: uremia and liver disease), CO (carbon monoxide) (source: CH₂Cl₂, elevated % COHb; diagnosis: indoor air pollution), chloroform (source: halogenated compounds), dichlorobenzene (source: halogenated compounds), diethylamine (source: choline; diagnosis: intestinal bacterial overgrowth), H (hydrogen) (source: intestines; diagnosis: lactose intolerance), isoprene (source: fatty acid; diagnosis: metabolic stress), methanethiol (source: methionine; diagnosis: intestinal bacterial overgrowth), methylethylketone (source: fatty acid; diagnosis: indoor air pollution/diet), O-toluidine (source: carcinoma metabolite; diagnosis: bronchogenic carcinoma), pentane sulfides and sulfides (source: lipid peroxidation; diagnosis: myocardial infarction), H₂S (source: metabolism; diagnosis: periodontal disease/ovulation), MeS (source: metabolism; diagnosis: cirrhosis), Me₂S (source: infection; diagnosis: trench mouth), αII-spectrin breakdown products and/or isoprostanes (source: cerebral spinal fluid, blood; diagnosis: traumatic or other brain injuries); prostate specific antigen (source: prostate cells; diagnosis: prostate cancer); and GLXA (source: gylcolipid in Chlamydia; diagnosis: Chlamydia).

The present invention can also detect any combination of the following SCEs: any nucleotide sequences provided in a genomic or cDNA library; any peptides in a phage displayed library; illicit, illegal, and/or controlled substances including drugs of abuse (i.e., amphetamines, analgesics, barbiturates, club drugs, cocaine, crack cocaine, depressants, designer drugs, ecstasy, Gamma Hydroxy Butyrate - GHB, hallucinogens, heroin/morphine, inhalants, ketamine, lysergic acid diethylamide - LSD, marijuana, methamphetamines, opiates/narcotics, phencyclidine - PCP, prescription drugs, psychedelics, Rohypnol, steroids, and stimulants); allergens (*i.e.,* pollen, spores, dander, peanuts, eggs, and shellfish); toxins (*i.e.*, mercury, lead, other heavy metals, and *Clostridium Difficile* toxin); carcinogens (i.e., acetaldehyde, beryllium compounds, chromium, dichlorodiphenyltrichloroethane (DDT), estrogens, N-methyl-N'-nitro-N-nitrosoguanidine (MNNG), and radon); infectious agents (*i.e*., *Bordettella bronchiseptica,* citrobacter, *Escherichia coli,* hepatitis viruses, herpes, immunodeficiency viruses, influenza virus, *Listeria,* micrococcus, mycobacterium, rabies virus, rhinovirus, rubella virus, *Salmonella,* and yellow fever virus), cell markers for diseases (*i.e*., T cell markers, B cell markers, myeloid/monocytic markers, maturity status markers for Leukemia, analplastic lymphoma, Hodgkins' disease; α-Fetoprotein (AFP) as an indicator of hepatocellular carcinoma and non-seminomatous testicular cancer; β2-Microglobulin (b2-M) as an indicator of active disease, cell turnover, tumor presence, and inflammatory diseases; and Beta Human Chorionic Gonadotropin (b HCG)) is a tumor marker for gestational trophoblastic diseases and germ cell tumors of the ovary or testis).

SCEs located in bodily fluids and that are not attached to cells are detected by certain embodiments of the invention. Such SCEs not only include biomarkers released by diseased cells but also include therapeutic and/or illicit drugs that are present in the body.

Examples of such unattached SCEs include, and are not limited to, the following: Alpha Fetoprotein (AFP), which is a useful tumor marker for the diagnosis and management of hepatocellular carcinoma and non-seminomatous testicular cancer; Beta2-Microglubulin (b2-M), high concentrations of which indicate active disease, cell turnover, tumor presence; the presence of inflammatory diseases (i.e., rheumatoid arthritis, systemic lupus erythematosus, Sjögren syndrome, Crohn's disease); or be a secondary indication of various lymphoproliferative diseases (leukemia, lymphoma, and multiple myeloma); Beta Human Chorionic Gonadotropin (b HCG), which is a tumor marker for gestational trophoblastic diseases, germ cell tumors of the ovary or testis, and cancers of the breast, lung, pancreas, stomach, kidney, and brain and is very helpful in assessing the efficacy of therapy in patients with testicular tumors; Carbohydrate antigen 19-9 (CA19-9), which is not organ specific but is a marker for a variety of adenocarcinomas (pancreatic, gastric, and hepatobiliary); and CA 125, which is found in most serous, endometrioid and clear cells carcinomas of the ovary.

Given the arrival of new technologies such as differential screening of phage displayed libraries to identify highly novel cell surface markers specific to different types of malignancies *(i.e.,* ovarian cancer), the utility of the nanostructure-based assemblies of the present invention to detect, notify, and monitor a wide variety of disease processes will markedly increase in the next decade. Table 1 illustrates certain new and older SCEs for key human maladies that can be detected using the present invention.

### Means for Detecting Specific Chemical Entities (SCEs)

A nanostructure-based assembly of the invention comprises a nanoparticle, which contains a means for detecting a target SCE, a surrogate marker, and, in certain embodiments, a payload. In a preferred embodiment, an SCE-detector is designed to detect a target SCE.

The SCE-detecting means of the invention can be selected from biosensors known to the skilled artisan. Such biosensors include naturally occurring and/or synthetic compounds having high specificity and sensitivity to chemical and/or biological compounds of interest. Suitable biosensors of the invention include, but are not limited to, antibodies, proteins, and aptamers.

Certain SCE-detectors of the invention are designed to alter the biological function of the target SCE. Other SCE-detectors can be designed to localize nanostructure-based assemblies within the vicinity of or into target cells for optimal release of payload (or surrogate marker). In related embodiments, the detecting means also has the capability of cellular localization (*i*.*e*., delivering the nanostructure-based assembly to a cancer cell) or subcellular localization (*i*.*e*., delivering the nanostructure-based assembly to a nucleus within a cancer cell).

The detecting means of the invention can allow for applications requiring specific SCE localization or immobilization (i.e., vectoring). See Langer, R., "Tissue Engineering," Mol Ther, 2:12-15 (2000). Detecting means including, for example, proteins, antibodies, peptides, RNA or DNA aptamers, cellular reporters or cellular ligands, can be attached to a nanoparticle surface to provide a means for vectoring the nanostructure-based assembly to a target SCE. Such SCE-detecting means may be covalently attached to the nanoparticle surface. In certain related embodiments, SCE-detecting means are attached to a nanoparticle surface via linker molecules. SCE-detecting means can also be attached to a nanoparticle surface by non-covalent linkage, for example, by absorption via hydrophobic binding or Van der Waals forces, hydrogen bonding, acid/base interactions, and electrostatic forces.

In one embodiment, the SCE-detector is an antibody specific to a target SCE. The antibody has a recognized structure that includes an immunoglobulin heavy and light chain. The heavy and light chains include an N-terminal variable region (V) and a C-terminal constant region (C). The heavy chain variable region is often referred to as "V_{H}" and the light chain variable region is referred to as "V_{L}". The V_{H} and V_{L} chains form a binding pocket that has been referred to as F(v). See generally Davis, 3: 537, Ann. Rev. of Immunology (1985); and Fundamental Immunology 3rd Ed., W. Paul Ed. Raven Press LTD. New York (1993). The shape of the binding pocket F(v) is complementary to that of the target SCE for efficient and specific binding. Generally, binding is due to weak, non-covalent bonds between the antibody and that of the target SCE. Antibodies can be attached to the nanoparticle using methods known to the skilled artisan.

Alternatively, recombinant bispecific antibody (bsFv) molecules can be used as an SCE-detector. In a preferred embodiment, bsFv molecules that bind a T-cell protein termed "CD3" and a TAA are used as an SCE-detector in accordance with the present invention. In related embodiments, bsFv molecules are used not only to specifically bind to a target SCE but also to facilitate an immune system response. See Jost, C. R. 33: 211, Mol. Immunol (1996); Lindhofer, H. et al. 88: 465 1, Blood (1996); Chapoval, A. I. et al. 4: 571, J. of Hematotherapy (1995).

With other embodiments of the present invention, the SCE-detecting means is in the form of an aptamer. Aptamers have the ability to recognize a broad range of targets, including small organic molecules as well as large proteins (Gold *et al., supra*.; Osborne and Ellington, "Nucleic acid selection and the challenge of combinatorial chemistry," Chem. Rev., 97:349-370 (1997)).

Aptamers derived from the SELEX™ methodology can be utilized in the present invention. The SELEX™ methodology enables the production of aptamers, each of which have a unique sequence and the property of binding specifically to a desired target compound or molecule. The SELEX™ methodology is based on the insight that nucleic acids have sufficient capacity for forming a variety of two- and three-dimensional structures and sufficient chemical versatility available within their monomers to act as ligands (form specific binding pairs) with virtually any chemical compound, whether monomeric or polymeric. Molecules of any size or composition can serve as targets. See also Jayasena, S., "Aptamers: An Emerging Class of Molecules That Rival Antibodies for Diagnostics," Clinical Chemistry, 45:9, 1628-1650 (1999).

Aptamers that can be used as SCE detectors in the present invention include those described in U.S. Patent No. 5,656,739 (hereinafter the '739 patent), which discloses the advantages of synthetic oligonucleotides as assembly templates. The '739 patent describes nucleic acids as particularly useful assembly templates because they can be selected to specifically bind nonoligonucleotide target molecules with high affinity (*e.g.,* Tuerk and Gold (1990), *supra*), and because they can hybridize by complementary base pairing. Both forms of recognition can be programmably synthesized in a single molecule or hybridized into a single discrete structure.

### Surrogate Markers

The surrogate marker of the invention can be any compound that can be identified in bodily fluids including radio-labeled or fluorescent compounds, compounds that change the color of bodily fluids for detection by the naked eye, or compounds that are readily identified in bodily fluids using sensor technology. Preferably, the detection of a surrogate marker in bodily fluids indicates the presence of target SCEs in the patient and enables the diagnosis of the disease associated with the target SCE.

For example, the surrogate marker can be a benzodiazepine or benzodiazepine metabolite that is detectable in urine. Benzodiazepines and their metabolites readily pass through the renal system into urine making benzodiazepines and substances with similar properties especially suitable as compliance markers. Examples of benzodiazepines or benzodiazepine metabolites that can be used in the invention include diazepam and alprazolam.

Additional surrogate markers contemplated herein include, without limitation, dimethyl sulfoxide (DMSO), acetaldehyde, acetophenone, anise, benzaldehyde, benzyl alcohol, benzyl cinnamate, cadinene, camphene, camphor, cinnamon, garlic, citronellal, cresol, cyclohexane, eucalyptol, and eugenol, eugenyl methyl ether. Such markers are particularly advantageous for use in detection in exhaled breath.

The surrogate markers of the invention also include additives that have been federally approved and categorized as GRAS ("generally recognized as safe"), which are available on a database maintained by the U.S. Food and Drug Administration Center for Food Safety and Applied Nutrition. Surrogate markers categorized as GRAS and are readily detectable in bodily fluids include, and are not limited to, sodium bisulfate, dioctyl sodium sulfosuccinate, polyglycerol polyricinoleic acid, calcium casein peptone-calcium phosphate, botanicals (i.e., chrysanthemum; licorice; jellywort, honeysuckle; lophatherum, mulberry leaf; frangipani; selfheal; sophora flower bud), ferrous bisglycinate chelate, seaweed-derived calcium, DHASCO (docosahexaenoic acid-rich single-cell oil) and ARASCO (arachidonic acid-rich single-cell oil), fructooligosaccharide, trehalose, gamma cyclodextrin, phytosterol esters, gum arabic, potassium bisulfate, stearyl alcohol, erythritol, D-tagatose, and mycoprotein.

### Payload Materials

The payload material, as contemplated herein, is a therapeutic bioactive agent used in the prevention, cure, or alleviation of a disease.

By way of example, one embodiment of the present invention uses nanoparticle-based assemblies that contain anti-oxidant genes (MnSOD, HO-1, and PON1) as the payload. Anti-oxidant genes are released in the presence of pro-atherogenic genes to enable treatment of atherosclerosis in a patient.

Payload materials of the invention can be selected from, but are not limited to, genetic material (*i.e.,* DNA); RNA; oligonucleotides; peptides; proteins (*i.e*., enzymes), chemotherapeutics (anti-cancer pharmaceuticals); antibiotics; antifungal agents; anesthetics; immunomodulators *(i.e.,* interferon, cyclosporine); antiinflammatory and other types of pain relieving agents; autonomic drugs; cardiovascular-renal drugs; endocrine drugs; hematopoietic growth factors; blood lipid lowering drugs; AIDS drugs; modulators of smooth muscle function; antileptics; psychoactive drugs; and drugs that act on the peripheral nerves, adrenergic receptors, cholinergic receptors, the skeletal muscles, the cardiovascular system, smooth muscles, the blood circulatory system, synoptic sites, neuroeffector junctional sites, endocrine and hormone systems, metabolic systems, the immunological system, the reproductive system, the skeletal system, autacoid systems, the alimentary and excretory systems, the histamine system, and the central nervous system. Suitable agents may be selected from, for example, proteins, enzymes, hormones, polynucleotides, nucleoproteins, polysaccharides, glycoproteins, lipoproteins, polypeptides, steroids, analgesics, local anesthetics, antibiotic agents, antiinflammatory corticosteroids, ocular drugs, and synthetic analogs of these species.

Examples of drugs which are delivered as payloads by nanostructure-based assemblies include, but are not limited to, prochlorperzine edisylate, ferrous sulfate, aminocaproic acid, mecamylamine hydrochloride, procainamide hydrochloride, amphetamine sulfate, methamphetamine hydrochloride, benzamphetamine hydrochloride, isoproterenol sulfate, phenmetrazine hydrochloride, bethanechol chloride, methacholine chloride, pilocarpine hydrochloride, atropine sulfate, scopolamine bromide, isopropamide iodide, tridihexethyl chloride, phenformin hydrochloride, methylphenidate hydrochloride, theophylline cholinate, cephalexin hydrochloride, diphenidol, meclizine hydrochloride, prochlorperazine maleate, phenoxybenzamine, thiethylperzine maleate, anisindone, diphenadione erthyrityl tetranitrate, digoxin, Intal (disodium cromoglycate), codeine, morphine, sodium salicylate, salicylic acid, meperidine hydrochloride (DEMEROL), chlophedianol hydrochloride, epinephrine, isoproterenol, salbutamol, terbutaline, ephedrine, aminophylline, acetylcysteine, sulfanilamide, sulfadiazine, tetracycline, rifampin (rifamycin), dihydrostreptomycin, p-aminosalicylic acid, hypoglycemics tolbutamide (ORINASE), prednisone, prednisolone, prednisolone metasulfobenzoate, chlorambucil, busulfan, alkaloids, antimetabolites, 6-mercaptopurine, thioguanine, 5-fluorouracil, hydroxyurea, isoflurophate, acetazolamide, methazolamide, bendroflumethiazide, chloropromaide, tolazamide, chlormadinone acetate, phenaglycodol, allopurinol, aluminum aspirin, methotrexate, acetyl sulfisoxazole, erthyromycin, hydrocortisone, hydrocorticosterone acetate, cortisone acetate, dexamethasone and its derivatives such as betamethasone, triamcinolone, methyltestosterone, 17-S-estradiol, ethinyl estradiol, ethinyl estradiol 3-methyl ether, 17-α-hydroxygrogesterone acetate, 19-norprogesterone, norgestrel, norethindrone, norethisterone, norethiederone, progesterone, norgesterone, norethynodrel, aspirin, indomethacin, naproxen, fenoprofen, sulindac, indoprofen, nitroglycerin, isosorbide dinitrate, propranolol, timolol, atenolol, alprenolol, crimetidine, clonidine, imipramine, levodopa, chlorpromazine, methyldopa, dihydroxyphenylanine, theophylline, calcium gluconate, ketoprofen, ibuprofen, cephalexin, erythromycin, haloperidol, zomepirac, ferrous lactate, vincamine, phenoxybenzamine, diltiazem, milrinone, mandol, quanbenz, hydrochlorothiazide, ranitidine, flurbiprofen, fenufen, fluprofen, tolmetin, alclofenac, mefenamic, flufenamic, difuinal, nimodipine, nitrendipine, nisoldipine, nicardipine, felodipine, lidoflazine, tiapamil, gallopamil, amlodipine, mioflazine, lisinolpril, enalapril, enalaprilat captopril, ramipril, famotidine, nizatidine, sucralfate, etintidine, tetratolol, minoxidil, chlordiazepoxide, diazepam, amitriptyline, and imipramine.

Further examples of payload materials are proteins and peptides which include, but are not limited to, bone morphogenic proteins, insulin, colchicines, glucagons, thyroid stimulating hormone, parathyroid and pituitary hormones, calcitonin, rennin, prolactin, conicotrophin, thyrotropic hormone, follicle stimulating hormone, chorionic gonadotropin, gonadotropin releasing hormone, bovine somatotropin, porcine somatotropin, oxytocin, vasopressin, GRF, somatostatin, lypressin, pancreozymin, luteinizing hormone, LHRH, LHRH agonists and antagonists, leuprolide, interferons such as interferon alpha-2a, interferon alpha-2b, and consensus interferon, interleukins, growth hormones such as human growth hormone and its derivatives such as methione-human growth hormone and desphenylalnine human growth hormone, bovine growth hormone and porcine growth hormone, fertility inhibitors such as prostaglandins, fertility promoters, growth factors such as insulin-like growth factor, coagulation factors, human pancreas hormone releasing factor, analogs and derivatives of these compounds, and pharmaceutically acceptable salts of these compounds, or their analogs or derivatives.

Additional payload materials which can be delivered by the nanostructure-based assemblies of the invention include, but are not limited to, chemotherapeutic agents such as carboplatin, cisplatin, paclitaxel, BCNU, vincristine, camptothecin, etopside, cytokines, ribozymes, interferons, oligonucleotides and oligonucleotide sequences that inhibit translation or transcription of tumor genes, functional derivatives of the foregoing, and generally known chemotherapeutic agents such as those described in U.S. Patent No. 5,651,986.

In certain embodiments, payload materials are therapeutic drugs as provided by the subject invention.

### Therapeutic Drugs

As contemplated herein, therapeutic drugs to be monitored in accordance with the subject invention include, but are not limited to, psychiatric drugs (i.e., antidepressants, anti-psychotics, anti-anxiety drugs, depressants), analgesics, stimulants, biological- response modifiers, NSAIDs, corticosteroids, DMARDs, anabolic steroids, antacids, antiarrhythmics, antibacterials, antibiotics, anticoagulants and thrombolytics, anticonvulsants, antidiarrheals, antiemetics, antihistamines, antihypertensives, anti-inflammatories, antineoplastics, antipyretics, antivirals, barbiturates, β-blockers, bronchodilators, cough suppressants, cytotoxics, decongestants, diuretics, expectorants, hormones, immunosuppressives, hypoglycemics, laxatives, muscle relaxants, sedatives, tranquilizers, and vitamins.

For example, the subject invention can effectively monitor concentrations of the following non-limiting list of therapeutic drugs in blood: drugs for the treatment of rheumatoid arthritis or symptoms thereof, systemic lupus erythematosus or symptoms thereof, degenerative arthritis, vasculitis, inflammatory diseases, angina, coronary artery disease, peripheral vascular disease; ulcerative colitis, and Crohn's disease; anti organ rejection drugs; antiepilepsy medication; and anti-anxiety drugs.

Therapeutic drugs whose concentration levels in blood can be monitored in accordance with the subject invention include, but are not limited to, the following: α-Hydroxy-Alprazolam; Acecainide (NAPA); Acetaminophen (Tylenol); Acetylmorphine; Acetylsalicylic Acid (as Salicylates); α-hydroxy-alprazolam; Alprazolam (Xanax); Amantadine (Symmetrel); Ambien (Zolpidem); Amikacin (Amikin); Amiodarone (Cordarone); Amitriptyline (Elavil) & Nortriptyline; Amobarbital (Amytal); Anafranil (Clomipramine) & Desmethylclomipramine; Ativan (Lorazepam); Aventyl (Norhiptyline); Benadryl (Dephenhydramine); Benziodiazepines; Benzoylecgonine; Benztropine (Cogentin); Bupivacaine (Marcaine); Bupropion (Wellbutrin) and Hydroxybupropion; Butabarbital (Butisol); Butalbital (Fiorinal) Carbamazepine (Tegretol); Cardizem (Diltiazem); Carisoprodol (Soma) & Meprobamate; and Celexa (Citalopram & Desmethylcitalopram).

Additional therapeutic drugs whose blood concentration levels can be monitored in accordance with the subject invention include Celontin (Methsuximide) (as desmethylmethsuximide); Centrax (Prazepam) (as Desmethyldiazepam); Chloramphenicol (Chloromycetin); Chlordiazepoxide; Chlorpromazine (Thorazine); Chlorpropamide (Diabinese); Clonazepam (Klonopin); Clorazepate (Tranxene); Clozapine; Cocaethylene; Codeine; Cogentin (Benztropine); Compazine (Prochlorperazine); Cordarone (Anuodarone); Coumadin (Warfarin); Cyclobenzaprine (Flexeril); Cyclosporine (Sandimmune); Cylert (Pemoline); Dalmane (Flurazepam) & Desalkylflurazepam; Darvocet; Darvon (Propoxyphene) & Norpropoxyphene; Demerol (Meperidine) & Normeperidine; Depakene (Valproic Acid); Depakote (Divalproex) (Measured as Valproic Acid); Desipramine (Norpramin); Desmethyldiazepam; Desyrel (Trazodone); Diazepam & Desmethyldiazepam; Diazepam (Valium) Desmethyldiazepam; Dieldrin; Digoxin (Lanoxin); Dilantin (Phenytoin); Disopyramide (Norpace); Dolophine (Methadone); Doriden (Glutethimide); Doxepin (Sinequan) and Desmethyldoxepin; Effexor (Venlafaxine); Ephedrine; Equanil (Meprobamate) Ethanol; Ethosuximide (Zarontin); Ethotoin (Peganone); Felbamate (Felbatol); Fentanyl (Innovar); Fioricet; Fipronil; Flunitrazepam (Rohypnol); Fluoxetine (Prozac) & Norfluoxetine; Fluphenazine (Prolixin); Fluvoxamine (Luvox); Gabapentin (Neurontin); Gamma-Hydroxybutyric Acid (GHB); Garamycin (Gentamicin); Gentamicin (Garamycin); Halazepam (Paxipam); Halcion (Triazolam); Haldol (Haloperidol); Hydrocodone (Hycodan); Hydroxyzine (Vistaril); Ibuprofen (Advil, Motrin, Nuprin, Rufen); Imipramine (Tofranil) and Desipramine; Inderal (Propranolol); Keppra (Levetiracetam); Ketamine; Lamotrigine (Lamictal); Lanoxin (Digoxin); Lidocaine (Xylocaine); Lindane (Gamma-BHC); Lithium; Lopressor (Metoprolol); Lorazepam (Ativan); and Ludiomil.

Blood level concentrations of the following therapeutic drugs that can be monitored in accordance with the subject invention include, but are not limited to, Maprotiline; Mebaral (Mephobarbital) & Phenobarbital; Mellaril (Thioridazine) & Mesoridazine; Mephenytoin (Mesantoin); Meprobamate (Miltown, Equanil); Mesantoin (Mephenytoin); Mesoridazine (Serentil); Methadone; Methotrexate (Mexate); Methsuximide (Celontin) (as desmethsuximide); Mexiletine (Mexitil); Midazolam (Versed); Mirtazapine (Remeron); Mogadone (Nitrazepam); Molindone (Moban); Morphine; Mysoline (Primidone) & Phenobarbital; NAPA & Procainamide (Pronestyl); NAPA (N-Acetyl- Procainamide); Navane (Thiothixene); Nebcin (Tobramycin); Nefazodone (Serzone); Nembutal (Pentobarbital); Nordiazepam; Olanzapine (Zyprexa); Opiates; Orinase (Tolbutamide); Oxazepam (Serax); Oxcarbazepine (Trileptal) as 10-Hydroxyoxcarbazepine; Oxycodone (Percodan); Oxymorphone (Numorphan); Pamelor (Nortriptyline); Paroxetine (Paxil); Paxil (Paroxetine); Paxipam (Halazepam); Peganone (Ethotoin); PEMA (Phenylethylmalonamide); Pentothal (Thiopental); Perphenazine (Trilafon); Phenergan (Promethazine); Phenothiazine; Phentermine; Phenylglyoxylic Acid; Procainamide (Pronestyl) & NAPA; Promazine (Sparine); Propafenone (Rythmol); Protriptyline (Vivactyl); Pseudoephedrine; Quetiapine (Seroquel); Restoril (Temazepam); Risperdal (Risperidone) and Hydroxyrisperidone; Secobarbital (Seconal); Sertraline (Zoloft) & Desmethylsertraline; Stelazine (Trifluoperazine); Surmontil (Trimipramine); Tocainide (Tonocard); and Topamax (Topiramate).

Therapeutic drugs of the subject invention can be formulated according to known methods for preparing pharmaceutically useful compositions. Formulations are described in a number of sources, which are well known and readily available to those skilled in the art. For example, Remington's Pharmaceutical Science (Martin EW [1995] Easton Pennsylvania, Mack Publishing Company, 19th ed.) describes formulations that can be used in connection with the subject invention. Formulations suitable for parenteral administration include, for example, aqueous sterile injection solutions, which may contain antioxidants, buffers, bacteriostats, and solutes, which render the formulation isotonic with the blood of the intended recipient; and aqueous and nonaqueous sterile suspensions, which may include suspending agents and thickening agents.

Formulations may be presented in unit-dose or multi-dose containers, for example sealed ampoules and vials, and may be stored in a freeze dried (lyophilized) condition requiring only the condition of the sterile liquid carrier, for example, water for injections, prior to use. Extemporaneous injection solutions and suspensions may be prepared from sterile powder, granules, tablets, *etc.* It should be understood that in addition to the ingredients particularly mentioned above, the formulations of the subject invention can include other agents conventional in the art having regard to the type of formulation in question.

Administration of a therapeutic drug, in accordance with the subject invention, can be accomplished by any suitable method and technique presently or prospectively known to those skilled in the art. In a preferred embodiment, a therapeutic drug is formulated in a patentable and easily consumed oral formulation such as a pill, lozenge, tablet, gum, beverage, etc.

According to the subject invention, a therapeutic drug can be delivered from a controlled supply means (i.e., nanostructure-base assembly, pill dispenser, IV bag, etc.). Upon delivery of the therapeutic drug to a patient, a sensor of the invention analyzes a patient's expired gases to detect at least one target marker of the therapeutic drug. Upon detection of the target marker, the concentration of the therapeutic drug in blood can be determined for use in deriving the appropriate dosage amount of the therapeutic drug to next be delivered to the patient. In one embodiment, a system controller utilizes the derived appropriate dosage based on exhaled breath analysis to dispense an appropriate dosage from the supply means to the patient.

Additional embodiments are also envisioned herein. Pulmonary delivery of medications is well known, especially for conditions such as asthma and chronic obstructive pulmonary disease. In these instances, medication *(i.e.,* corticosteroids, bronchodilators, anticholenergics, etc.) is often nebulized or aerosolized and inhaled through the mouth directly into the lungs. This allows delivery directly to the affected organ (the lungs) and reduces side effects common with enteral (oral) delivery. Metered dose inhalers (MDIs) or nebulizers are commonly used to deliver medication by this route. Recently dry powder inhalers have become increasingly popular, as they do not require the use of propellants such as CFCs. Propellants have been implicated in worsening asthma attacks, as well as depleting the ozone layer. Dry power inhalers are also being used for drugs that were previously given only by other routes, such as insulin, peptides, and hormones.

Olfactory markers can be added to these delivery systems as well. Since the devices are designed to deliver medication by the pulmonary route, the sensor array can be incorporated into the device and the patient need only exhale back through the device for documentation to occur.

Lastly, devices are available to deliver medication by the intranasal route. This route is often used for patients with viral infections or allergic rhinitis, but is being increasing used to deliver peptides and hormones as well. Again, it would be simple to incorporate a sensor array into these devices, or the patient can exhale through the nose for detection by a marker sensing system.

### Therapeutic Drug Markers

In accordance with the present invention, therapeutic drug markers useful as an indication of therapeutic drug concentration in blood include the following olfactory markers, without limitation: dimethyl sulfoxide (DMSO), acetaldehyde, acetophenone, trans-Anethole (1-methoxy-4-propenyl benzene) (anise), benzaldehyde (benzoic aldehyde), benzyl alcohol, benzyl cinnamate, cadinene, camphene, camphor, cinnamaldehyde (3-phenylpropenal), garlic, citronellal, cresol, cyclohexane, eucalyptol, and eugenol, eugenyl methyl ether; butyl isobutyrate (n-butyl 2, methyl propanoate) (pineapple); citral (2-trans-3,7-dimethyl-2,6-actadiene-1-al); menthol (1-methyl-4-isopropylcyclohexane-3-ol); and α-Pinene (2,6,6-trimethylbicyclo-(3,1,1)-2-heptene). These markers are preferred since they are used in the food industry as flavor ingredients and are permitted by the Food and Drug Administration. As indicated above, olfactory markers for use in the present invention can be selected from a vast number of available compounds (see Fenaroli's Handbook of Flavor Ingredients, 4th edition, CRC Press, 2001) and use of such other applicable markers is contemplated herein.

The markers of the invention also include additives that have been federally approved and categorized as GRAS ("generally recognized as safe"), which are available on a database maintained by the U.S. Food and Drug Administration Center for Food Safety and Applied Nutrition. Markers categorized as GRAS that are readily detectable in exhaled breath include, but are not limited to, sodium bisulfate, dioctyl sodium sulfosuccinate, polyglycerol polyricinoleic acid, calcium casein peptone-calcium phosphate, botanicals *(i.e.,* chrysanthemum; licorice; jellywort, honeysuckle; lophatherum, mulberry leaf; frangipani; selfheal; sophora flower bud), ferrous bisglycinate chelate, seaweed-derived calcium, DHASCO (docosahexaenoic acid-rich single-cell oil) and ARASCO (arachidonic acid-rich single-cell oil), fructooligosaccharide, trehalose, gamma cyclodextrin, phytosterol esters, gum arabic, potassium bisulfate, stearyl alcohol, erythritol, D-tagatose, and mycoprotein.

As described above, therapeutic drug markers are detected by their physical and/or chemical properties, which does not preclude using the desired therapeutic drug itself as its own marker. Therapeutic drug markers, as contemplated herein, also include products and compounds that are administered to enhance detection using sensors of the invention. Moreover, therapeutic drug markers can include a variety of products or compounds that are added to a desired therapeutic drug regimen to enhance differentiation in detection/quantification. Generally, in accordance with the present invention, therapeutic drug markers are poorly soluble in water, which enhances their volatility and detection in the breath.

According to the subject invention, upon administering a therapeutic drug (wherein the therapeutic drug is the marker) or upon concurrent administration of a therapeutic drug and marker, marker detection can occur under several circumstances. In one example where the drug is administered orally, the marker can "coat" or persist in the mouth, esophagus and/or stomach upon ingestion and be detected with exhalation (similar to the taste or flavor that remains in the mouth after eating a breath mint).

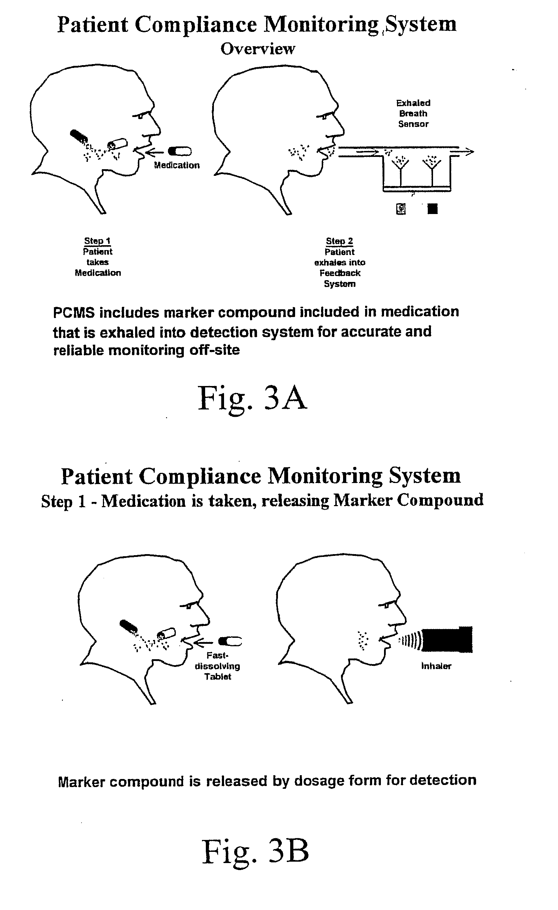

In a second instance, such as illustrated in Figures 3A and 3B, where the drug (and marker) is administered orally, the drug may react in the mouth or stomach with acid or enzymes to produce or liberate the marker that can then be detected upon exhalation. Thirdly, the drug and/or marker can be absorbed in the gastrointestinal tract and be excreted in the lungs *(i.e.* alcohol is rapidly absorbed and detected with a Breathalyzer). Generally, a therapeutic drug marker of the invention provides a means for determining the pharmacodynamics and pharmacokinetics of the drug.

In one embodiment, a therapeutic drug marker is concurrently administered with a therapeutic drug (*i.e*., marker is provided in a pharmaceutically acceptable carrier - marker in medication coating composed of rapidly dissolving glucose and/or sucrose. In a preferred embodiment, the therapeutic drug is provided in the form of a pill, whose coating includes at least one marker in air-flocculated sugar crystals. This would stimulate salivation and serve to spread the marker around the oral cavity, enhancing the lifetime in the cavity. Since the throat and esophagus could also be coated with the marker as the medication is ingested, detection of the marker is further enhanced.

Thus, when a drug is administered to a patient, the preferred embodiment of the invention detects and quantifies a therapeutic drug marker almost immediately in the exhaled breath of the patient (or possibly by requesting the patient to deliberately produce a burp) using a sensor (i.e., electronic nose). Certain drug compositions might not be detectable in the exhaled breath. Others might have a coating to prevent the medication from dissolving in the stomach. In both instances, as an alternate embodiment, a non-toxic olfactory marker *(i.e.,* volatile organic vapors) can be added to the pharmaceutically acceptable carrier (i.e., the coating of a pill, in a separate fast dissolving compartment in the pill, or solution, if the drug is administered in liquid or suspension form) to provide a means for identifying/quantifying the marker in exhaled breath and thus determine the drug concentration in blood.

Preferably the marker will coat the oral cavity or esophagus or stomach for a short while and be exhaled in the breath (or in a burp). For drugs administered in the form of pills, capsules, and fast-dissolving tablets, the markers can be applied as coatings or physically combined or added to therapeutic drug. Markers can also be included with therapeutic drugs that are administered in liquid form (i.e., syrups, via inhalers, or other dosing means).

The markers of the invention could be used for indicating specific drugs or for a class of drugs. For example, a patient may be taking an anti-depressant (tricyclics such as nortriptyline), antibiotic, an antihypertensive agent *(i.e.,* clonidine), pain medication, and an anti-reflux drug. One marker could be used for antibiotics as a class, or for subclasses of antibiotics, such as erythromycins. Another marker could be used for antihypertensives as a class, or for specific subclasses of antihypertensives, such as calcium channel blockers. The same would be true for the anti-reflux drug. Furthermore, combinations of marker substances could be used allowing a rather small number of markers to specifically identify a large number of medications.

### Pharmacodynamics and Pharmacokinetics of Therapeutic Drugs

When a therapeutic drug is administered to a patient in accordance with the subject invention, there are many factors which effect drug pharmacodynamics and pharmacokinetics. For example, drug affinity (i.e., degree of attraction between a drug and a target receptor in the patient body), drug distribution (i.e., binding of drug to proteins circulating in the blood, absorption of drug into fat), drug metabolism and elimination (i.e., renal clearance), or existence of a drug in a "free" form may affect drug pharmacodynamics and pharmacokinetics in a patient.

A drug bound to protein or absorbed into fat does not produce a desired pharmacological effect and exists in equilibrium with unbound drug. Numerous factors, including competition for binding sites on the protein from other drugs, the amount of fat in the body, and the amount of protein produced, determine the equilibrium between bound and unbound drug.

An unbound drug can participate directly in the pharmacological effect or be metabolized into a drug that produces a desired effect. Metabolism of the active drug often leads to its removal from the bloodstream and termination of its effect. The drug effect can also be terminated by the excretion of the free drug. Free drug or a metabolite can be excreted in the urine or the digestive tract or in exhaled breath. The concentration in the blood (or plasma or serum) of such therapeutic drugs is related to the clinical effect of the agent.

As described above, blood concentration testing for a therapeutic drug may or may not provide an accurate indication of the effect of the therapeutic drug on a patient, since measurement of blood concentration does not account for the quantity of drug bound to protein or membranes, or the interaction and competition between drugs. For this reason, it would be advantageous to measure only the free drug in the plasma. The concentration of free drug in plasma is usually low and requires sophisticated and expensive analytical techniques for measurement. By contrast, the therapeutic drug marker that is present in breath, in accordance with the subject invention, is an indication of the concentration of free drug in blood. Thus, the systems and methods of the subject invention provide an effective indicator of the actual concentration of free drug responsible for pharmacokinetic effect.

Further, testing blood directly (i.e., drawing blood for sample analysis) is invasive, time consuming, expensive, and prone to inaccuracies. In contrast, by analyzing therapeutic drug markers in patient exhaled breath, the systems and methods of the subject invention are non-invasive, speedy, and accurate. When a therapeutic drug marker is excreted in the breath, the concentration in expired breath is proportional to the free therapeutic drug concentration in the blood and, thus, indicative of the rate of drug absorption, distribution, metabolism, and/or elimination.

In certain embodiments, a metabolite may act as a therapeutic drug marker to be measured in exhaled breath where the metabolite is a product of the active drug. As long as there is equilibrium between the active drug and a metabolite excreted in the breath, the activity of the active drug can be analyzed in accordance with the subject invention.

The method of the present invention takes into account such proportional concentrations and allows for the determination of the rate of absorption, distribution, metabolism, and elimination of a therapeutic drug by measuring concentration of unbound substances, markers, and/or active metabolites associated with the drug in a patient's breath. The proper dosing regimen can thus be determined therefrom.

### Sensor Technology

Sensor technology is used by the present invention to detect the presence of a surrogate marker in a bodily fluid sample. The detection of a surrogate marker signifies the presence and/or quantity of a target SCE. In certain embodiments, the detection of a surrogate marker can also indicate release of payload/treatment.

A sensor of the invention can be selected from a wide variety of sensor technology including, but is not limited to, those described in U.S. Patent Nos. 6,010,459; 5,081,871; 5,042,501; 4,202,352; 5,971,937, and 4,734,777, "artificial" or electronic" noses or tongues, semiconductor gas sensor technology, conductive polymer gas sensor technology, surface acoustic wave gas sensor technology, immunoassays, metal-insulator-metal ensemble (MIME) sensors, cross-reactive optical microsensor arrays, fluorescent polymer films, and surface enhanced raman spectroscopy (SERS).

The present invention contemplates using sensor technology based on surface acoustic wave (SAW) sensors. These sensors oscillate at high frequencies and respond to perturbations proportional to the mass load of certain molecules. This occurs in the vapor phase on the sensor surface. The resulting frequency shift is detected and measured by a computer. Usually, an array of sensors (such as 4-6 sensors) is used, each coated with a different chemoselective polymer that selectively binds and/or absorbs vapors of specific classes of molecules. The resulting array, or "signature" identifies specific compounds. Sensitivity of the arrays is dependent upon the homogeneity and thickness of the polymer coating.

Surface-acoustic-wave (SAW) gas-sensors generally include a substrate with piezoelectric characteristics covered by a polymer coating, which is able to selectively absorb a surrogate marker. The variation of the resulting mass leads to a variation of its resonant frequency. This type of sensor provides very good mass-volume measures of the surrogate markers. In the SAW device, the surrogate marker is used to propagate a surface acoustic wave between sets of interdigitated electrodes. The chemoselective material is coated on the surface of the transducer. When a surrogate marker interacts with the chemoselective material coated on the substrate, the interaction results in a change in the SAW properties, such as the amplitude or velocity of the propagated wave. The detectable change in the characteristics of the wave indicates the presence and concentration of the surrogate marker (and corresponding target SCE).

A SAW vapor sensing device has been disclosed in which a layer of antibodies are attached to a surface of the SAW sensor (see Stubbs, DD et al., "Investigation of Cocaine Plumes Using Surface Acoustic Wave Immunoassay Sensors," Anal. Chem., 75:6231-6235 (2003)). When a target antigen reacts with an antibody, the acoustic velocity is altered, causing an oscillator frequency of the SAW to shift to a different value. The subject invention contemplates usage of such SAW devices, as well as those SAW sensing devices in which aptamers (including indicator aptamers), molecular beacons, and other known biosensors are utilized to coat a surface of the SAW sensor.

Certain embodiments use known SAW devices described in numerous patents and publications, including U.S. Patent Nos. 4,312,228 and 4,895,017, and Groves W.A. et al., Analyzing organic vapors in exhaled breath using surface acoustic wave sensor arra with preconcentration: - Selection and characterization of the preconcentrator adsorbent," Analytica Chimica Acta, 371:131-143 (1988).

Other types of chemical sensors known in the art that use chemoselective coating appliable to the operation of the present invention include bulk acoustic wave (BAV) devices, plate acoustic wave devices, interdigitated microelectrode (IME) devices, optical waveguide (OW) devices, electrochemical sensors, and electrically conducting sensors.

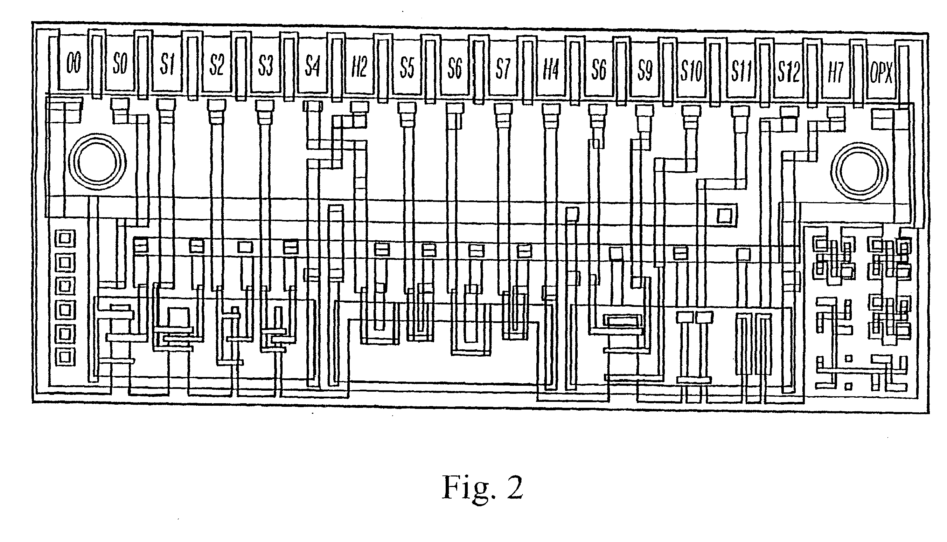

In another embodiment, the invention uses fluid sensor technology, such as commercial devices known as "artificial noses," "electronic noses," or "electronic tongues." These devices are capable of qualitative and/or quantitative analysis of simple or complex gases, vapors, odors, liquids, or solutions. A number of patents and patent applications which describe fluid sensor technology include the following: U.S. Patent Nos. 5,945,069; 5,918,257; 5,891,398; 5,830,412; 5,783,154; 5,756,879; 5,605,612; 5,252,292; 5,145,645; 5,071,770; 5,034,192; 4,938,928; and 4,99 2,244; and U.S. Patent Application No. 2001/0050228. Certain sensitive, commercial off-the-shelf electronic noses, such as those provided by Cyrano Sciences, Inc. ("CSI") (*i.e*., CSI's portable Electronic Nose and CSI's Nose-Chip™ integrated circuit for odor-sensing - U.S. Patent No. 5,945,069), are preferably used in the present invention to detect the presence of detectable (surrogate and/or therapeutic drug) markers in bodily fluid samples (Figure 2).

Other embodiments of the present invention use sensor technology selected from semiconductive gas sensors; mass spectrometers; and IR, UV, visible, or fluorescence spectrophotometers. With these sensors, a surrogate or therapeutic drug marker changes the electrical properties of the semiconductors by making their electrical resistance vary, and the measurement of these alternatives allows the determination of the concentration of detectable markers present in the sample. The methods and apparatus used for detecting (surrogate and/or therapeutic drug) markers generally have a brief detection time of a few seconds.

Additional recent sensor technologies included in the present invention include apparatus having conductive-polymer gas-sensors ("polymeric"), aptamer biosensors, and amplifying fluorescent polymer (AFP) sensors.

Conductive-polymer gas-sensors (also referred to as "chemoresistors") are coated with a film sensitive to the molecules of certain detectable markers. On contact with the molecules, the electric resistance of the sensors change and the measurement of the variation of this resistance enable the concentration of the detected substance (i.e., surrogate marker and corresponding target SCE or therapeutic drug marker and associated therapeutic drug concentration in blood) to be determined. An advantage of this type of sensor is that it functions at temperatures close to ambient. Different sensitivities for detecting different detectable markers can be obtained by modifying or choosing an alternate conductive polymer.

Polymeric gas sensors can be built into an array of sensors, where each sensor responds to different gases and augment the selectivity of the (surrogate or therapeutic drug) marker.

Aptamer-based biosensors can be utilized in the present invention for detecting the presence of surrogate and/or therapeutic drug markers in bodily fluid samples. Aptamer biosensors are resonant oscillating quartz sensors that can detect minute changes in resonance frequencies due to modulations of mass of the oscillating system, which results from a binding or dissociation event.

Similarly, amplifying fluorescent polymer (AFP) sensors may be utilized in the present invention for detecting the presence of detectable markers in bodily fluid samples. AFP sensors are extremely sensitive and highly selective chemosensors that use amplifying fluorescent polymers. When vapors bind to thin films of the polymers, the fluorescence of the film decreases. A single molecule binding event quenches the fluorescence of many polymer repeat units, resulting in an amplification of the quenching. The binding of target markers to the film is reversible, therefore the films can be reused.

In accordance with the present invention, competitive binding immunoassays can be used to test a bodily fluid sample for the presence of (surrogate and/or therapeutic drug) markers. Immunoassay tests generally include an absorbent, fibrous strip having one or more reagents incorporated at specific zones on the strip. The bodily fluid sample is deposited on the strip and by capillary action the sample will migrate along the strip, entering specific reagent zones in which a chemical reaction may take place. At least one reagent is included which manifests a detectable response, for example a color change, in the presence of a minimal amount of a marker of interest. Patents that describe immunoassay technology include the following: U.S. Patent Nos. 5,262,333 and 5,573,955.

Other embodiments of the present invention use flow cytometers to analyze bodily fluid samples for target compounds. Flow cytometry is a technique that is used to determine certain physical and chemical properties of microscopic biological particles by sensing certain optical properties of the particles. To do so, the particles are arranged in single file using hydrodynamic focusing within a sheath fluid. The particles are then individually interrogated by a light beam. Each particle scatters the light beam and produces a scatter profile. The scatter profile is often identified by measuring the light intensity at different scatter angles. Certain physical and/or chemical properties of each particle can then be determined from the scatter profile. Patents that describe flow cytometry technology include the following: U.S. Patent Nos. 6,597,438; 6,097,485; 6,007,775; and 5,716,852.

The sensor of the present invention might include integrated circuits (chips) manufactured in a modified vacuum chamber for Pulsed Laser Deposition of polymer coatings. It will operate the simultaneous thin-film deposition wave detection and obtain optimum conditions for high sensitivity of SAW sensors. The morphology and microstructure of biosensor coatings will be characterized as a function of process parameters.

Where the bodily fluid is exhaled breath, the sensor used in the subject invention may be modified so that patients can exhale directly onto the sensor, without needing a breath sampling apparatus. For example, a mouthpiece or nosepiece will be provided for interfacing a patient with the device to readily transmit the exhaled breath to the sensor (See, *i.e.,* U.S. Patent No. 5,042,501). In a related embodiment, wherein the sensor is connected to a neural network, the output from the neural network is similar when the same patient exhales directly into the device and when the exhaled gases are allowed to dry before the sensor samples them.

The humidity in the exhaled gases represents a problem for certain electronic nose devices (albeit not SAW sensors) that only work with "dry" gases. When using such humidity sensitive devices, the present invention may adapt such electronic nose technology so that a patient can exhale directly into the device with a means to dehumidify the samples. This is accomplished by including a commercial dehumidifier or a heat moisture exchanger (HME), a device designed to prevent desiccation of the airway during ventilation with dry gases.

Alternatively, the patient may exhale through their nose, which is an anatomical, physiological dehumidifier to prevent dehydration during normal respiration. Alternatively, the sensor device can be fitted with a preconcentrator, which has some of the properties of a GC column. The gas sample is routed through the preconcentrator before being passed over the sensor array. By heating and volatilizing the gases, humidity is removed and the marker being measured can be separated from potential interferents.

Preferably, in operation, the sensor will be used to identify a baseline spectrum for the patient prior to drug administration, if necessary. This will prove beneficial for the detection of more than one therapeutic drug if the patient receives more than one drug at a time and possible interference from different foods and odors in the stomach, mouth, esophagus and lungs.

### Reporting Means and Data Monitor/Analyzer

Results from sensor technology analysis of bodily fluid samples are optionally provided to the user (or patient) via a reporting means. In one embodiment, sensor technology includes the reporting means. Contemplated reporting means include a computer processor linked to the sensor technology in which electronic or printed results can be provided. Alternatively, the reporting means can include a digital display panel, transportable read/write magnetic media such as computer disks and tapes_which can be transported to and read on another machine, and printers such as thermal, laser or ink-jet printers for the production of a printed report.

The reporting means can provide the results to the user (or patient) via facsimile, electronic mail, mail or courier service, or any other means of safely and securely sending the report to the patient. Interactive reporting means are also contemplated by the present invention, such as an interactive voice response system, interactive computer-based reporting system, interactive telephone touch-tone system, or other similar system. The report provided to the user (or patient) may take many forms, including a summary of analyses performed over a particular period of time or detailed information regarding a particular bodily fluid sample analysis. Results may also be used to populate a financial database for billing the patient, or for populating a laboratory database or a statistical database.

A data monitor/analyzer can compare a pattern of response to previously measured and characterized responses from known markers. The matching of those patterns can be performed using a number of techniques, including neural networks. By comparing the analog output from each of the 32 polymers to a "blank" or control, for example, a neural network can establish a pattern that is unique to that marker and subsequently learns to recognize that marker. The particular resistor geometries are selected to optimize the desired response to the target marker being sensed. The sensor of the subject invention is preferably a self-calibrating polymer system suitable for detecting and quantifying markers in gas phase biological solutions to assess and/or monitor a variety of therapeutic drug markers simultaneously.

According to the subject invention, the sensor can include a computer that communicates therewith, which can also notify the medical staff and/or the patient as to any irregularities in dosing, dangerous drug interactions, and the like. This system will enable determination as to whether a patient has been administered a pharmacologically effective amount of a therapeutic drug. The device could also alert the patient (or user) as to time intervals and/or dosage of therapeutic drug to be administered. Accordingly, it is contemplated herein that a sensor of the subject invention can be portable.

### Bodily Fluid Samples

After administration of the nanostructure-based assembly and/or therapeutic drug to a patient, a sample of bodily fluid is collected from the patient for analysis. The bodily fluid sample is analyzed for the presence of the surrogate marker and/or therapeutic drug marker. Detection of the surrogate marker indicates the presence of the SCE in the patient and consequently, allows for the non-invasive, point-of-care disease diagnosis.

Where a payload and/or therapeutic drug marker are provided in a nanostructure-based assembly, substantially simultaneous treatment and diagnosis of a disease are provided as well as monitoring of therapeutic drug blood concentration. Specifically, detection and/or quantification of surrogate markers in exhaled breath enable disease diagnosis and notification of localized treatment delivery. Detection of therapeutic drug markers indicates the concentration of the therapeutic drug in blood.

According to the invention, a sample of bodily fluid includes, but is not limited to, exhaled breath (including cough, sneeze), blood, urine, sweat, mucous, semen, bile, feces, saliva, lymph fluid, blood plasma, amniotic fluid, glandular fluid, sputum, and cerebral spinal fluid. Preferred embodiments of the invention analyze samples of exhaled breath.

Generally, the exhalation gas stream comprises sequences or stages. At the beginning of exhalation there is an initial stage, the gas representative thereof coming from an anatomically inactive (deadspace) part of the respiratory system, in other words, from the mouth and upper respiratory tracts. This is followed by a plateau stage. Early in the plateau stage, the gas is a mixture of deadspace and metabolically active gases. The last portion of the exhaled breath comprises nothing but deep lung gas, so-called alveolar gas. This gas, which comes from the alveoli, is termed end-tidal gas.

In a preferred embodiment, the exhaled breath sample is collected at end-tidal breathing. Technology similar to that used for end-tidal carbon dioxide monitoring can be used to determine when the sample is collected. Known methods for airway pressure measurements afford another means of collecting samples at the appropriate phase of the respiratory cycle. Single or multiple samples collected by the known side stream method are preferable, but if sensor acquisition time is reduced, in-line sampling may be used. In the former, samples are collected through an adapter at the proximal end of an endotracheal (ET) tube and drawn through thin bore tubing to a sensor of the subject invention.

Depending on the sample size and sensor response time, exhaled gas may be collected on successive cycles. With in-line sampling, a sensor of the subject invention is placed proximal to the ET tube directly in the gas stream. Alternatively to sample end-tidal gas, samples can be taken throughout the exhalation phase of respiration and an average value determined and correlated with blood concentration.

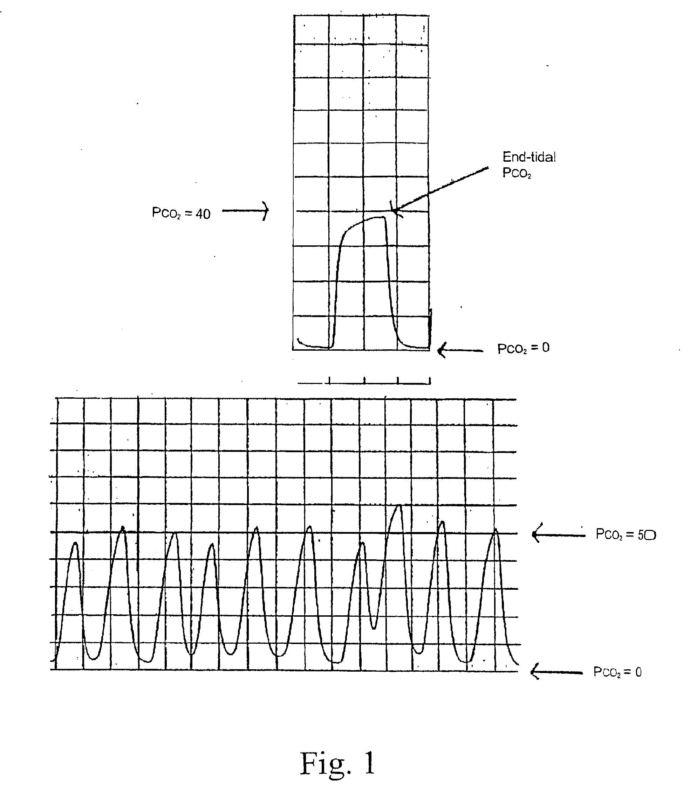

Referring now to Figure 1, the upper frame demonstrates a capnogram of a single respiratory cycle. For accurate blood level correlation, samples are taken at the point labeled "end-tidal PCO₂" which reflects the CO₂ concentration in the lung. The lower frame shows a capnogram of several breaths from a patient with obstructive lung disease. Again the end-tidal sample correlated best with blood concentration.

In one embodiment, a VaporLab™ brand instrument is used to collect and analyze exhaled breath samples. The VaporLab™ instrument is a hand-held, battery powered SAW-based chemical vapor identification instrument suitable for detecting components in exhaled breath samples in accordance with the present invention. This instrument is sensitive to volatile and semi-volatile compounds using a high-stability SAW sensor array that provides orthogonal vapor responses for greater accuracy and discrimination. In a related embodiment, this instrument communicates with computers to provide enhanced pattern analysis and report generation. In a preferred embodiment, this instrument includes neural networks for "training" purposes, i.e., to remember chemical vapor signature patterns for fast, "on-the-fly" analysis.

In another embodiment, samples are collected at the distal end of an ET tube through a tube with a separate sampling port. This may improve sampling by allowing a larger sample during each respiratory cycle.

In certain instances, the concentration of a therapeutic drug in a patient body is regulated by the amount of the drug administered over a given time period and the rate at which the agent is eliminated from the body (metabolism). The present invention provides the steps of administering a therapeutic drug to a patient and analyzing patient exhaled breath for concentration of therapeutic drug markers such as unbound substances, active metabolites, or inactive metabolites associated with the therapeutic drug, after a suitable time period. In certain embodiments of the subject invention, the marker concentration indicates a characteristic of metabolism of the drug in the patient.

Methods of the subject invention may further include the use of a flow sensor to detect starting and completion of exhalation. The method further includes providing results from the analysis and communicating to the user or patient the blood concentration of the therapeutic drug. In a preferred embodiment, results from analysis can be communicated immediately upon sampling exhaled gases.

In certain embodiments, the subject invention enables the immediate monitoring of therapeutic drug levels in a patient's blood stream. As contemplated herein, immediate monitoring refers to sampling and analysis of exhaled gases from a patient for target markers substantially completely within a short time period following administration of a therapeutic drug (*i.e.,* generally within a few minutes to about 24 hours).

Alternatively, in certain instances, a specific period of time must progress before a therapeutic drug concentration level in the blood stream can be detected. Accordingly, a system and/or method of the invention can be provided to a patient taking a therapeutic drug for intermittent or continuous monitoring of therapeutic drug concentrations in the blood stream. In certain embodiments, the monitoring system and method of the subject invention can be administered to a patient taking a therapeutic drug on an hourly, daily, weekly, monthly, or even annual basis. Further, additional monitoring can be administered to a patient when an additional therapeutic drug is prescribed.

Moreover, a CPU may be provided as a data processing/control unit for automatically detecting the signal from the flow sensor to control sampling of exhaled breath. The CPU may further provide to the user/patient the appropriate dosage of the therapeutic drug to be delivered based on analysis of trends in therapeutic drug blood concentration.

Depending on the mode of therapeutic drug administration, the present invention provides means for automatically adjusting and administering the appropriate dosage of a therapeutic drug, based on blood concentration levels, to a patient. In certain embodiments, a CPU is provided for analysis and control of dosage adjusting and administering means. In one embodiment in which a therapeutic drug is delivered intravenously, an infusion pump is used, wherein the CPU provides analysis and control of the infusion pump.

Concentration in the blood of therapeutic drug markers, as measured by breath analysis in accordance with the present invention, may indicate when the patient is receiving a high dose (*i.e*., toxic dose), a low dose (*i.e*., ineffective dose), or effective (i.e., appropriate) dose of the therapeutic drug. Even if there is wide variation in the metabolism or response to the therapeutic drug, knowledge of the exhaled breath concentration allows the user to know if the drug is accumulating in the blood, possibly leading to dangerously toxic levels of the drug, or that the concentration is falling, possibly leading to an inadequate dose of the drug. Monitoring changes in therapeutic drug blood concentration in accordance with the subject invention are, therefore, useful.

In another embodiment, the exhalation air is measured for marker concentration either continuously or periodically. From the exhalation air is extracted at least one measured marker concentration value. Numerous types of breath sampling apparatuses can be used to carry out the method of the present invention.

In one embodiment, the breath sampling apparatus includes a conventional flow channel through which exhalation air flows. The flow channel is provided with a sensor of the subject invention for measuring marker concentration. Furthermore, necessary output elements may be included with the breath sampling apparatus for delivering at least a measured concentration result to the user, if necessary.

An alarm mechanism may also be provided. An instrument of similar type is shown in Figures 1 and 2 of U.S. Patent No. 5,971,937 incorporated herein by reference.

In another embodiment, once the level of concentration is measured, it is given numerical value (for example, 50 on a scale of 1 to 100). Should the concentration fall below that value, the new value would be indicative of a decrease in concentration. Should the concentration increase beyond that value, the new value would be indicative of an increase in concentration. This numerical scale would allow for easier monitoring of changes in concentration. The numerical scale would also allow for easier translation into control signals for alarms, outputs, charting, and control of external devices (e.g., infusion pump). The upper and lower limits could be set to indicate thresholds such as from ineffective to dangerous therapeutic drug levels.

Compositions containing nanostructure-based assemblies in accordance with the present invention can be administered utilizing methods known to the skilled artisan. In one aspect of the invention, the compositions are formulated in admixture with a pharmaceutically acceptable carrier and optionally, with other therapeutic and/or prophylactic ingredients.

In general, it is preferable to administer a pharmaceutical composition of the invention in orally or nasally (i.e., inhalation) administrable form, but formulations may be administered via parenteral, intravenous, intramuscular, transdermal *(i.e.,* topical), buccal, subcutaneous, transmucosal, suppository or other route. Intravenous and intramuscular compositions are preferably administered in sterile saline. One of ordinary skill in the art may modify the compositions of the invention within the teachings of the specification to provide numerous formulations for a particular route of administration without rendering the compositions of the present invention unstable or compromising its therapeutic activity. In particular, a modification of a desired compound to render it more soluble in water or other vehicle, for example, may be easily accomplished by routine modification (salt formulation, esterification).

According to the present invention, compositions can be delivered to the patient parenterally *(i.e.,* intravenously, intramuscularly). For such forms of administration, the compositions can be formulated into solutions or suspensions, or in lyophilized forms for conversion into solutions or suspensions before use. Sterile water, physiological saline (*i.e.*, phosphate buffered saline (PBS)) can be used conveniently as the pharmaceutically acceptable carriers or diluents. Conventional solvents, surfactants, stabilizers, pH balancing buffers, anti-bacterial agents, chelating agents, and antioxidants can all be used in the these formulations, including but not limited to acetates, citrates or phosphates buffers, sodium chloride, dextrose, fixed oils, glycerine, polyethylene glycol, propylene glycol, benzyl alcohol, methyl parabens, ascorbic acid, sodium bisulfite, and the like. These formulation can be stored in any conventional containers such as vials, ampoules, and syringes.

Sterile injectable solutions of the compositions of the invention can be prepared by incorporating the nanostructure-based assemblies in required amounts in an appropriate solvent with one or a combination of ingredients as required, followed by sterilization. Generally, dispersions are prepared by incorporating the nanostructure-based assemblies into a sterile vehicle that contains a basic dispersion medium, and the other required ingredients. Preparation of sterile powders for sterile injectable solutions include vacuum drying and freeze-drying that yield a powder containing the active ingredient and any desired ingredients to form a sterile solution.

The compositions of the invention can also be delivered orally in enclosed gelatin capsules or compressed tablets. Capsules and tablets can be prepared in any conventional techniques. For example, the active compounds can be incorporated into a formulation, which includes pharmaceutically acceptable carriers such as excipients (i.e., starch, lactose), binders (i.e., gelatin, cellulose, gum tragacanth), disintegrating agents (i.e., alginate, Primogel, and corn starch), lubricants (i.e., magnesium stearate, silicon dioxide), and sweetening or flavoring agents (*i.e*., glucose, sucrose, saccharin, methyl salicylate, and peppermint). Various coatings can also be prepared for the capsules and tablets to modify the flavors, tastes, colors, and shapes of the capsules and tablets. In addition, liquid carriers such as fatty oil can also be included in capsules

The nanostructure-based assemblies of the invention can be added to a medical formulation by homogeneously mixing them throughout the formulation or solution of the therapeutic medication. Alternatively, the nanostructure-based assemblies are formed as a film or coating on a tablet or capsule containing the therapeutic medication. If more than one medication has been prescribed, a separate first and/or second detectable marker can be used in association with each medication. Preferably the first and/or second markers of the invention have biological half-lives of between 24 and 48 hours so that they will appear in a sample of bodily fluids taken from the patient.

### Remote Communication System

A further embodiment of the invention includes a communications device in the home (or other remote location) that will be interfaced to sensor technology. The home communications device will be able to transmit immediately or at prescribed intervals directly or over a standard telephone line (or other communication transmittal means such as via satellite communication) the data collected by the data monitor/analyzer device. The communication of the data will allow the user *(i.e.,* physician) to be able to remotely verify if the appropriate dosage of a therapeutic drug is being administered to the patient. The data transmitted from the home can also be downloaded to a computer where the drug blood levels are stored in a database, and any deviations outside of pharmacological efficacy would be automatically flagged (i.e., alarm) so that a user *(i.e.,* patient, physician, nurse) could appropriately adjust the drug dosage per suggestions provided by a computer processing unit connected to the sensor or per dosage suggestions provided by health care personnel (i.e., physician).

### Example 1-Systems and Methods for Testing Heroin Use

In one embodiment, a patient suffering from heroin addiction is administered a composition comprising nanoparticle-based assemblies of the invention. The nanoparticle-based assemblies are designed to detect the drug heroin. In one embodiment, the nanoparticle-based assemblies contain a nanoparticle, a surrogate marker, and an SCE-detector. Preferably, the SCE-detector is an aptamer that is designed to be specific for heroin (heroin-aptamer). The heroin-aptamer and the surrogate marker (heroin-surrogate marker) are attached to a surface of the nanoparticle.

In a preferred embodiment, the heroin-aptamer is attached to an end-cap of a hollow nanoparticle that contains therein the heroin-surrogate marker. The heroin-aptamer is designed so that upon interaction with heroin, the end-cap is released from the nanoparticle to release the heroin-surrogate marker. The heroin-surrogate marker is readily detectable in bodily fluid samples taken from the patient.

To test for heroin use, the nanoparticle-based assemblies are administered to the patient and then a sample of the patient's bodily fluid (i.e., urine, breath, blood) is acquired. Where heroin is present in the patient, the heroin interacts with the heroin-aptamer and "uncaps" the nanoparticle, thus releasing the heroin-surrogate marker for identification in the bodily fluid sample. Any one of a number of previously disclosed sensor technologies is then used to detect the heroin-surrogate marker, where the heroin-surrogate marker indicates presence of heroin in the patient's body.

### Example 2-Treatment of Atherosclerosis

In another embodiment of the invention, a patient suffering from atherosclerosis is administered a composition comprising nanoparticle-based assemblies to diagnose and treat atherosclerosis. The nanoparticle-based assembly comprises a nanoparticle; a surrogate marker; a payload; and an SCE-detector. Treatment of atherosclerosis (payload) comprises anti-oxidant genes (MnSOD, HO-1 and PON1) that utilize the patient's own hormonal changes to offset atherosclerotic disease progression. The SCE-detector is designed to detect biomarkers of atherosclerosis (i.e., ICAM-1, VCAM-1, or LOX-1). ICAM-1, VCAM-1, and LOX-1 are pro-atherogenic genes in human coronary endothelial cells that are regulated by cytokine levels (IL1, TNF, IL-6).

Once the SCE-detector is in the presence of an atherosclerosis biomarker, it causes the release of the anti-oxidant genes and the surrogate marker. The antioxidant genes not only alter the development of atherosclerosis but also afford cytoprotective treatment to vascular endothelium to prevent the development of atherosclerosis. The surrogate marker is an indicator in bodily fluid samples that pro-atherogenic biomarkers are present in the patient as well as an indicator that antioxidant genes have been administered to the patient.

### Example 3-Diagnosis and Treatment of Glycogen Storage Disorder

Glycogen is readily detectable in bodily fluids (i.e., blood) using a nanoparticle-based assembly of the invention. According to the present invention, the nanoparticle-based assembly comprises a nanoparticle, a surrogate marker, and an SCE-detector that is designed to bind to the glycogen and to act upon the glycogen in a fashion similar to muscle phosphorylase to safely break down glycogen. Binding of the SCE-detector to glycogen causes the release of the surrogate marker for detection. Thus, with the present invention, it is possible to not only diagnose a specific disease/condition in a patient but also to treat it and ensure patient compliance with the treatment regimen. In addition, the method of the present invention can evaluate pharmacodynamics and pharmacokinetics for drug interventions in individuals.

### Example 4-Assessment of Blood and Diagnosis/Treatment of Blood-Based Diseases

In one embodiment, the nanostructure-based assemblies of the invention can be used to differentiate and signal types of blood cells and their concentrations in the patient. For example, levels of red blood cells (RBCs), white blood cells (WBCs), and platelets can be assessed using the systems and methods of the invention to diagnose and/or treat hematopoiesis abnormalities such as leukemia or assess changes in cellular contect (e.g., RBC content).

Accordingly, the subject invention is useful in diagnosing and/or treating blood-based diseases or disorders including, without limitation, hemorrhagic diathesis (i.e., hemophilia, von Willebrand disease, Alexander's disease, Telfer's disease, Owren's parahemophilia, prothrombin deficiency); non-hemorrhagiparous coagulopathies (i.e., Fletcher factor deficiency, Flaujeac factor deficiency); thrombophilic coagulopathies (*i.e*., Ratnoff's disease, thrombomodulin deficiency); thrombocytopenia; anemias; and alterations in white blood cells (*i.e.,* Pelger-Huët anomaly (PHA); Chediak-Higashi syndrome (CHS); Hegglin-May anomaly (HMA)). Example 5-Estimation of free blood propofol concentration during intravenous administration by measurement of exhaled breath propofol with a SAW-based sensor system of the invention

Propofol, an intravenous anesthetic agent, is frequently administered by continuous infusion to provide sedation to patients in the intensive care unit (ICU). Propofol is extremely lipophilic and also binds strongly to proteins and red blood cells. It is estimated that only 1-3% of propofol is free in plasma. It is this free fraction of propofol that is responsible for the desired therapeutic effect.

Often during a clinical procedure, it is desirable to periodically stop the propofol infusion to perform neurological examinations on patients, particularly those who have suffered a brain injury. Unfortunately, depending on the pharmacodynamics of propofol in an individual patient, the free blood concentration can be greater or less than that estimated by population pharmacodynamics and pharmacokinetics. This can lead to inadequate sedation, which may result in agitation and additional brain insult, or to accumulation of propofol in adipose tissue, resulting in prolonged sedation or even anesthesia, preventing adequate neurological examination.

The subject invention overcomes these deficiencies in the use of propofol. By continuously monitoring the end-tidal exhaled breath propofol concentration, an infusion pump can be programmed and regulated to maintain a precise exhaled breath, and thus, blood concentration of propofol. This will allow the healthcare provider to maintain the patient in a precise plane of sedation or anesthesia and overcome many of the complications related to using propofol for long periods of time where it might accumulate in adipose tissue and/or compete for binding sites on proteins and red blood cells.

### Example 6-Estimation of antibiotic blood concentrations using exhaled breath measurements as a surrogate

Patients requiring intravenous antibiotics for serious infections often require frequent blood sampling to obtain antibiotic concentrations. Often "peak" and "trough" levels are drawn to insure that the blood concentration of drug is adequate just prior to giving the next dose. Inadequate blood levels can predispose to bacteria developing drug resistance. A sensor for analyzing antibiotic markers in exhaled breath can be calibrated against a peak and trough level and for all subsequent measurements for use as a surrogate for measuring blood antibiotic levels and to subsequently direct therapy.

### Example 7-Exhaled breath anti-seizure medication levels as a surrogate for blood concentration.

Patients taking anti-seizure medications require frequent testing and analysis of blood samples to determine the concentration of the medication in their blood. Many anti-seizure medications have a narrow therapeutic range and low blood levels can lead to an increased frequency of seizures, while high levels can lead to significant toxicity. A sensor for detecting in exhaled breath anti-seizure medication markers can be calibrated against the blood anti-seizure medication concentration and used to monitor blood levels without the patient having to visit the physician or a laboratory to have blood drawn. The exhaled breath concentrations would alert the physician when the drug dose needs to be adjusted.

It should be understood that the examples and embodiments described herein are for illustrative purposes only and that various modifications or changes in light thereof will be suggested to persons skilled in the art and are to be included within the spirit and purview of this application and the scope of the appended claims. Specifically, the marker detection method of the present invention is intended to cover detection not only through the exhalation by a patient with a device utilizing electronic nose technology, but also other suitable technologies, such as gas chromatography, transcutaneous/transdermal detection, semiconductive gas sensors, mass spectrometers, IR or UV or visible or fluorescence spectrophotometers.

All patents, patent applications, provisional applications, and publications referred to or cited herein, or from which a claim for benefit of priority has been made, are incorporated by reference in their entirety to the extent they are not inconsistent with the explicit teachings of this specification

## Claims

1. A method of monitoring a patient during administration of at least one therapeutic drug, said method comprising:
administering to the patient at least one therapeutic drug;
exposing at least one sensor to expired gases from the patient;
detecting one or more target markers from the therapeutic drug with said sensor.

2. The method of claim 1, wherein said target marker is the therapeutic drug.

3. The method of claim 1, wherein said target marker is a metabolite of the therapeutic drug indicative of the therapeutic drug.

4. The method of claim 1, wherein said target marker is selected from a group consisting of dimethyl sulfoxide (DMSO), acetaldehyde, acetophenone, trans-Anethole (1-methoxy-4-propenyl benzene) (anise), benzaldehyde (benzoic aldehyde), benzyl alcohol, benzyl cinnamate, cadinene, camphene, camphor, cinnamaldehyde (3-phenylpropenal), garlic, citronellal, cresol, cyclohexane, eucalyptol, and eugenol, eugenyl methyl ether; butyl isobutyrate (n-butyl 2, methyl propanoate) (pineapple); citral (2-trans-3,7-dimethyl-2,6-actadiene-1-al); menthol (1-methyl-4-isopropylcyclohexane-3-ol); and α-pinene (2,6,6-trimethylbicyclo-(3,1,1)-2-heptene).

5. The method of claim 1, wherein at least one therapeutic drug is administered to the patient orally.

6. The method of claim 1, wherein at least one therapeutic drug is delivered intravenously.

7. The method of claim 1, wherein the detecting step comprises detecting both presence and concentration of the target marker to determine at least one therapeutic drug concentration in blood.

8. The method of claim 7, further comprising assigning a numerical value to the concentration as analyzed upon reaching a level of therapeutic effect of said therapeutic drug in said patient and, thereafter, assigning higher or lower values to the concentration based on its relative changes.

9. The method of claim 8, further comprising monitoring the concentration by monitoring changes in said value and adjusting administration of the therapeutic drug to maintain a desired therapeutic effect.

10. The method of claim 7, further comprising determining an appropriate dosage of at least one therapeutic drug based on the concentration of at least one target marker detected in said expired gases.

11. The method of claim 1, wherein the steps are repeated periodically to monitor pharmacodynamics and pharmacokinetics of at least one therapeutic drug over time.

12. The method of claim 1, wherein at least one therapeutic drug is for depression.

13. The method of claim 1, wherein at least one therapeutic drug is for analgesia.

14. The method of claim 1, wherein at least one therapeutic drug is selected for the treatment of a condition selected from group consisting of rheumatoid arthritis, systemic lupus erythematosus, angina, coronary artery disease, peripheral vascular disease, ulcerative colitis, Crohn's disease, organ rejection, epilepsy, anxiety, degenerative arthritis, vasculitis, and inflammation.

15. The method of claim 1, wherein the detecting is continuous.

16. The method of claim 1, wherein the detecting is periodic.

17. The method of claim 1, wherein at least one therapeutic drug is selected from the group consisting of: α-Hydroxy-Alprazolam; Acecainide (NAPA); Acetaminophen (Tylenol); Acetylmorphine; Acetylsalicylic Acid (as Salicylates); α-hydroxy-alprazolam; Alprazolam (Xanax); Amantadine (Symmetrel); Ambien (Zolpidem); Amikacin (Amikin); Amiodarone (Cordarone); Amitriptyline (Elavil) & Nortriptyline; Amobarbital (Amytal); Anafranil (Clomipramine) & Desmethylclomipramine; Ativan (Lorazepam); Aventyl (Nortriptyline); Benadryl (Dephenhydramine); Benziodiazepines; Benzoylecgonine; Benztropine (Cogentin); Bupivacaine (Marcaine); Bupropion (Wellbutrin) and Hydroxybupropion; Butabarbital (Butisol); Butalbital (Fiorinal) Carbamazepine (Tegretol); Cardizem (Diltiazem); Carisoprodol (Soma) & Meprobamate; and Celexa (Citalopram & Desmethylcitalopram).

18. The method of claim 1, wherein at least one therapeutic drug is selected from the group consisting of: Celontin (Methsuximide) (as desmethylmethsuximide); Centrax (Prazepam) (as Desmethyldiazepam); Chloramphenicol (Chloromycetin); Chlordiazepoxide; Chlorpromazine (Thorazine); Chlorpropamide (Diabinese); Clonazepam (Klonopin); Clorazepate (Tranxene); Clozapine; Cocaethylene; Codeine; Cogentin (Benztropine); Compazine (Prochlorperazine); Cordarone (Amiodarone); Coumadin (Warfarin); Cyclobenzaprine (Flexeril); Cyclosporine (Sandimmune); Cylert (Pemoline); Dalmane (Flurazepam) & Desalkylflurazepam; Darvocet; Darvon (Propoxyphene) & Norpropoxyphene; Demerol (Meperidine) & Normeperidine; Depakene (Valproic Acid); Depakote (Divalproex) (Measured as Valproic Acid); Desipramine (Norpramin); Desmethyldiazepam; Desyrel (Trazodone); Diazepam & Desmethyldiazepam; Diazepam (Valium) Desmethyldiazepam; Dieldrin; Digoxin (Lanoxin); Dilantin (Phenytoin); Disopyramide (Norpace); Dolophine (Methadone); Doriden (Glutethimide); Doxepin (Sinequan) and Desmethyldoxepin; Effexor (Venlafaxine); Ephedrine; Equanil (Meprobamate) Ethanol; Ethosuximide (Zarontin); Ethotoin (Peganone); Felbamate (Felbatol); Fentanyl (Innovar); Fioricet; Fipronil; Flunitrazepam (Rohypnol); Fluoxetine (Prozac) & Norfluoxetine; Fluphenazine (Prolixin); Fluvoxamine (Luvox); Gabapentin (Neurontin); Gamma-Hydroxybutyric Acid (GHB); Garamycin (Gentamicin); Gentamicin (Garamycin); Halazepam (Paxipam); Halcion (Triazolam); Haldol (Haloperidol); Hydrocodone (Hycodan); Hydroxyzine (Vistaril); Ibuprofen (Advil, Motrin, Nuprin, Rufen); Imipramine (Tofranil) and Desipramine; Inderal (Propranolol); Keppra (Levetiracetam); Ketamine; Lamotrigine (Lamictal); Lanoxin (Digoxin); Lidocaine (Xylocaine); Lindane (Gamma-BHC); Lithium; Lopressor (Metoprolol); Lorazepam (Ativan); and I,udiomil.

19. The method of claim 1, wherein at least one therapeutic drug is selected from the group consisting of: Maprotiline; Mebaral (Mephobarbital) & Phenobarbital; Mellaril (Thioridazine) & Mesoridazine; Mephenytoin (Mesantoin); Meprobamate (Miltown, Equanil); Mesantoin (Mephenytoin); Mesoridazine (Serentil); Methadone; Methotrexate (Mexate); Methsuximide (Celontin) (as desmethsuximide); Mexiletine (Mexitil); Midazolam (Versed); Mirtazapine (Remeron); Mogadone (Nitrazepam); Molindone (Moban); Morphine; Mysoline (Primidone) & Phenobarbital; NAPA & Procainamide (Pronestyl); NAPA (N-Acetyl- Procainamide): Navane (Thiothixene); Nebcin (Tobramycin); Nefazodone (Serzone); Nembutal (Pentobarbital); Nordiazepam; Olanzapine (Zyprexa); Opiates; Orinase (Tolbutamide); Oxazepam (Serax); Oxcarbazepine (Trileptal) as 10-Hydroxyoxcarbazepine; Oxycodone (Percodan); Oxymorphone (Numorphan); Pamelor (Nortriptyline); Paroxetine (Paxil); Paxil (Paroxetine); Paxipam (Halazepam); Peganone (Ethotoin); PEMA (Phenylethylmalonamide); Pentothal (Thiopental); Perphenazine (Trilafon); Phenergan (Promethazine); Phenothiazine; Phentermine; Phenylglyoxylic Acid; Procainamide (Pronestyl) & NAPA; Promazine (Sparine); Propafenone (Rythmol); Protriptyline (Vivactyl); Pseudoephedrine; Quetiapine (Seroquel); Restoril (Temazepam); Risperdal (Risperidone) and Hydroxyrisperidone; Secobarbital (Seconal); Sertraline (Zoloft) & Desmethylsertraline; Stelazine (Trifluoperazine); Surmontil (Trimipramine); Tocainide (Tonocard); and Topamax (Topiramate).

20. The method of claim 1, wherein said sensor is selected from the group consisting of: metal-insulator-metal ensemple (MIME) sensors, cross-reactive optical microsensor arrays, fluorescent polymer films, surface enhanced raman spectroscopy (SERS), diode lasers, selected ion flow tubes, metal oxide sensors (MOS), bulk acoustic wave (BAW) sensors, colorimetric tubes, infrared spectroscopy, gas chromatography, semiconductive gas sensor technology; mass spectrometers, gluorescent spectrophotometers, conductive polymer gas sensor technology; aptamer sensor technology; amplifying fluorescent polymer (AFP) sensor technology; or surface acoustic wave gas sensor technology.

21. The method of claim 20, wherein the sensor technology produces a unique electronic fingerprint to characterize the detection and concentration of said at least one target marker.

22. The method of claim 1, further comprising the step of recording data from said sensor.

23. The method of claim 1, further comprising the step of transmitting data from said sensor.

24. The method of claim 1, further comprising comparing at least one target marker detected with a predetermined signature profile.

25. The method of claim 1, further comprising capturing a sample of expired gases prior to exposing said sensor to expired gases.

26. The method of claim 1, further comprising dehumidifying expired gases prior to exposing said sensor to expired gases.

27. The method of claim 1, further comprising exposing said sensor to expired gases during exhalation of the patient's breath.

28. The method of claim 1, further comprising assigning a numerical value to the concentration as analyzed upon reaching a level of anesthetic effect in said patient and, thereafter, assigning higher or lower values to the concentration based on its relative changes.

29. The method of claim 1, wherein said sensor is portable.

30. The method of claim 1, wherein at least one therapeutic drug is a psychiatric drug.

31. The method of claim 30, wherein at least one therapeutic drug is selected from the group consisting of antidepressants, anti-psychotics, anti-anxiety drugs, and depressants.

32. A therapeutic drug delivery and monitoring system for delivering an appropriate dosage of the therapeutic drug to a patient:
at least one therapeutic drug supply having a controller for controlling the amount of therapeutic drug provided by the supply to the patient;
an expired gas sensor for analyzing the patient's breath for the presence and concentration of at least one target marker indicative of therapeutic drug concentrations in the patient's bloodstream, and for sending a signal regarding the concentration of the therapeutic drug in the patient's bloodstream; and
a system controller connected to the therapeutic drug supply, which receives and analyzes the signal from the sensor and controls the amount of therapeutic drug administered to the patient based on the signal.

33. The system of claim 32, wherein the expired gas sensor comprise a sensor for analyzing the gas for concentration of at least one target marker indicative of the therapeutic drug concentration in the patient's bloodstream and a processor for calculating the pharmacodynamic and pharmacokinetic effect of the therapeutic drug based on the concentration of the therapeutic drug.

34. The system of claim 33, wherein the sensor is selected from the group consisting of: metal-insulator-metal ensemple (MIME) sensors, cross-reactive optical microsensor arrays, fluorescent polymer films, surface enhanced raman spectroscopy (SERS), diode lasers, selected ion flow tubes, metal oxide sensors (MOS), bulk acoustic wave (BAW) sensors, colorimetric tubes, infrared spectroscopy, gas chromatography, semiconductive gas sensor technology; mass spectrometers, gluorescent spectrophotometers, conductive polymer gas sensor technology; aptamer sensor technology; amplifying fluorescent polymer (AFP) sensor technology; or surface acoustic wave gas sensor technology.
